(19) Europäisches Patentamt
European Patent Office
Office européen des brevets

(11) EP 1 236 800 A2

(12) EUROPEAN PATENT APPLICATION

(43) Date of publication:
**04.09.2002 Bulletin 2002/36**

(21) Application number: **02290515.2**

(22) Date of filing: **01.03.2002**

(51) Int Cl.[7]: **C12N 15/21**, C07K 14/56, C12Q 1/68, C07K 16/24, A61K 38/21, A61K 31/7088, A61K 39/395

(84) Designated Contracting States:
**AT BE CH CY DE DK ES FI FR GB GR IE IT LI LU MC NL PT SE TR**
Designated Extension States:
**AL LT LV MK RO SI**

(30) Priority: **01.03.2001 FR 0102843**

(71) Applicant: **GenOdyssee**
**91974 Courtaboeuf (FR)**

(72) Inventor: **Escary, Jean-Louis**
**78150 Le Chesnay (FR)**

(74) Representative: **Rinuy, Santarelli**
**14, avenue de la Grande Armée,**
**B.P. 237**
**75822 Paris Cédex 17 (FR)**

# (54) Nucleic acids encoding interferon alpha 2, containing single nucleotide polymorphisms and methods of use thereof

(57) The present invention relates to new polynucleotides deriving from the nucleotide sequence of the IFNα-2 gene and comprising new SNP(s). The present invention also concerns new polypeptides derived from the natural wild-type protein IFNα-2 and comprising a mutation caused by the SNP(s) of the invention. The present invention is also directed to the therapeutic uses of said new polynucleotides and polypeptides.

EP 1 236 800 A2

## Description

The present invention relates to new polynucleotides deriving from the nucleotide sequence of the IFNα-2 gene and comprising new SNPs, new polypeptides derived from the natural IFNα-2 protein and comprising mutations caused by these SNPs as well as their therapeutic uses.

## PRIOR ART

The interferon alpha-2 (IFNα-2) gene is described in the following publications:

- Olopade Ol., Bohlander Sk. "Mapping of the shortest region of overlap of deletions of the short arm of chromosome 9 associated with human neoplasia." Genomics. 1992 Oct; 14(2):437-43;
- Ezekowitz Ra., Mulliken Jb., "Interferon alpha-2a therapy for life-threatening hemangiomas of infancy" N. Engl. J. Med. 1992 May 28; 326(22):1456-63.;
- Dithmar S., Rusciano D., "Neoadjuvant interferon alpha-2b treatment in a murine model for metastatic ocular melanoma: a preliminary study" Arch. Ophthalmol. 2000 Aug; 118(8): 1085-9.

The nucleotide sequence of this gene is accessible under accession number J00207, V11834 in the GenBank database.

The IFNα are known for their cellular antiproliferative effects and their involvements in antiviral and antiparasitic responses.

The IFNα are also known to inhibit the expression of several other cytokines at the level of the hematopoietic stem cells, as well as to inhibit the cellular proliferation of certain tumors.

The IFNα are also known to reduce the expression of EGF receptors in renal carcinomas, to inhibit the expression of certain mitochondrial genes, to inhibit the proliferation of fibroblasts, monocytes and B lymphocytes, especially in vitro, and to block the synthesis of antibodies by B lymphocytes.

The IFNα are also known to induce the expression of tumors specific antigens on the surface of tumor cells and also to induce the genes placed under the control of promoter regions of the ISRE type (Interferon-Stimulated Response Element) by acting on the specific transcription factors of these ISRE.

It is known that the IFNα are involved in different disorders and/or human diseases, including but not limited to the different cancers such as carcinomas, melanomas, lymphomas, leukemias and cancers of the liver, neck, head and kidneys, cardiovascular diseases, metabolic diseases such as those that are not connected with the immune system like, for example, obesity, infectious diseases such as hepatitis B and C and AIDS, pneumonias, ulcerative colitis, diseases of the central nervous system like, for example, Alzheimer's disease, schizophrenia and depression, the rejection of tissue or organ grafts, healing of wounds, anemia in dialyzed patients, allergies, asthma, multiple sclerosis, osteoporosis, psoriasis, rheumatoid arthritis, Crohn's disease, autoimmune diseases and disorders, gastrointestinal disorders, and disorders connected with chemotherapy treatments.

The IFNα are particularly used for the treatment of certain leukemias, metastasized renal carcinomas as well as tumors that appear following an immunodeficiency, such as Kaposi's sarcoma in the case of AIDS. The IFNα are also effective against other types of tumors and against certain viral infections. The IFNα are also recognized by the FDA (Food and Drug Administration) for the treatment of genital warts or venereal diseases.

However, the IFNα and in particular IFNα-2, have numerous side effects when they are used in pharmaceutical compositions, such as reactions of acute hypersensitivity (urticaria, bronchoconstriction, anaphylactic shock etc.), cardiac arrythmias, low blood pressure, epileptic seizures, problems with thyroid functions, flu-like syndromes (fevers, sweats, myalgias) etc.

Furthermore, patients treated with IFNα can develop antibodies to these molecules, which neutralize and thus decrease their effectiveness.

The present invention is drawn to new polypeptide and new polynucleotide analogs to the IFNα-2 gene and its corresponding protein capable of having a different functionality from the natural wild-type IFNα-2 protein. In particular, certain of these new polypeptides and new polynucleotides have a cellular antiproliferative activity significantly inhibited by comparison with the natural wild-type IFNα-2.

These new polypeptides and polynucleotides can notably be used to treat or prevent the disorders or diseases previously mentioned and avoid all or part of the disadvantages, which are tied to them.

## THE INVENTION

The present invention is directed to new polynucleotides that differ from the nucleotide sequence of the reference wild-type IFNα-2 gene, in that it comprises one or more SNPs (Single Nucleotide Polymorphism).

The nucleotide sequence SEQ ID N°1 of the human reference wild-type IFNα-2 gene is composed of 1733 nucleotides and comprises a coding sequence of 567 nucleotides, from nucleotide 511 (start codon) to nucleotide 1077 (stop codon).

The applicant has identified 9 SNPs in the nucleotide sequence of the reference wild-type IFNα-2 gene.

These 9 SNPs are the following: 96-100del(aattt), t110c, 139-144del(acttta), t338a, t363c, c427t, c527a, g1023a, c1047t.

It is understood, in the sense of the present invention, that the numbering corresponds to the positioning of the SNPs previously defined and is relative to the nucleotide sequence shown in SEQ ID N°1.

The letters a, t, c and g correspond respectively to the nitrogenous bases adenine, thymine, cytosine and guanine.

The first letter corresponds to the wild-type nucleotide, whereas the last letter corresponds to the mutated nucleotide.

Thus, for example, the SNP c527a corresponds to a mutation of the nucleotide cytosine (c) at position 527 of the nucleotide sequence SEQ ID N°1 of the reference wild-type IFNα-2 gene into an adenine (a) and the SNP 96-100del(aattt) corresponds to a mutation in which the 5 nucleotides aattt from positions 96 to 100 of the nucleotide sequence SEQ ID N°1 of the reference wild-type IFNα-2 gene have been deleted.

These SNPs were identified by the applicant using the determination process described in applicant's patent applications FR 00 22894, filed December 6, 2000 and US 10/010 749, filed on December 6, 2001, cited here by way of reference.

The process described in these patent applications permits the identification of one (or several) preexisting SNP(s) in at least one individual from a random population of individuals.

In the scope of the present invention, two fragments of the nucleotide sequence of the IFNα-2 gene, one of which comprises the complete coding sequence, were isolated from different individuals in a population of individuals chosen in a random manner.

Sequencing of these fragments was then carried out on certain of these samples having a heteroduplex profile (that is a profile different from that of the reference wild-type IFNα-2 gene sequence) after analysis by DHPLC ("Denaturing-High Performance Liquid Chromatography").

The fragment sequenced in this way was then compared to the nucleotide sequence of the fragment of the reference wild-type IFNα-2 gene and the SNPs in conformity with the invention identified.

Thus, the SNPs are natural and each of them is present in certain individuals of the world population.

The reference wild-type IFNα-2 gene codes for an immature protein of 188 amino acids, corresponding to the amino acid sequence SEQ ID N°2, that will be converted to a mature protein of 165 amino acids by cleavage of the signal peptide that includes the first 23 amino acids.

Each of the coding SNPs of the invention, namely: c527a and g1023a, causes modifications, at the level of the amino acid sequence, of the protein encoded by the nucleotide sequence of the IFNα-2 gene.

These modifications in the amino acid sequence are the following:

The SNP c527a causes a mutation of the amino acid alanine (A) at position 6 in the immature protein encoded by the IFNα-2 gene, corresponding to the amino acid sequence SEQ ID N°2, in aspartic acid (D) and is not present in the mature protein since it belongs to the signal sequence. In the description of the present invention the mutation encoded by this SNP will also be called A6D.

The SNP c527a affects an amino acid residue located in the signal sequence of the protein. This signal sequence contains all the information necessary for the proper targeting of the mature protein. Thus, the SNP c527a may affect the final localization of the mature protein.

The SNP g1023a causes a mutation of the amino acid methionine (M) at position 171 in the immature protein encoded by the IFNα-2 gene, corresponding to the amino acid sequence SEQ ID N°2, in isoleucine (I) and at position 148 of the mature protein. In the description of the present invention, the terminology M148I and M171I will be used to refer to the mutation encoded by this SNP according to whether one refers respectively to the mature protein or to the immature protein, respectively.

The SNP g1023a causes modifications of the spatial conformation of the polypeptides in conformity with the invention compared to the polypeptide encoded by the nucleotide sequence of the reference wild-type IFNα-2 gene.

The modifications in the spatial conformation can be observed by computational molecular modeling, according to methods that are well known to a person skilled in the art, making use of, for example, the modeling tools *de novo* (for example, SEQFOLD/MSI), homology (for example, MODELER/MSI), minimization of the force field (for example, DISCOVER, DELPHI/MSI) and/or molecular dynamics (for example, CFF/MSI).

One example of such modelings is given hereinafter in the experimental part.

Computational molecular modeling permits the observation that the mutation M148I on the mature mutated protein causes a change in the lateral chain near the point of mutation, on helices A and E of IFNα-2. The mutated lateral chain I148 is engaged into a salt bridge with the lateral chain E141, which causes some changes in spatial conformation of the mature mutated protein. Whereas, in the three dimensional conformation of the wild-type IFNα-2, the R144 lateral chain is oriented towards the interior of the molecule, the R 144 lateral chain is oriented towards the exterior in the mature mutated protein. Likewise, in the mature mutated protein, the R22 and E141 lateral chains are displaced. Thus, the mutated protein possesses a three-dimensional conformation different from the natural wild-type IFNα-2 protein.

Thus, computational molecular modeling permits the prediction that the presence of the amino acid methionine at position 148 involves a significant modification of the structure and of the function of the reference wild-type IFNα-2 protein.

Other SNPs in conformity with the invention, namely: 96-100del(aattt), t110c, 139-144del(acttta), t338a, t363c, c427t, c1047t, do not involve modification of the protein encoded by the nucleotide sequence of the IFNα-2 gene at the level of the amino acid sequence SEQ ID N°2.

The SNP c1047t is silent and the SNPs 96-100del(aattt), t110c, 139-144del(acttta), t338a, t363c, c427t are non-coding.

Genotyping of the polynucleotides in conformity with the invention can be carried out in such a fashion as to determine the allelic frequency of these polynucleotides in a population. Six examples of genotyping are given, hereinafter, in the experimental part.

The determination of the functionality of the polypeptides of the invention can also be carried out by a test of their biological activity.

In this regard, it is possible to measure, for example, the antiproliferative effect of polypeptides in conformity with the invention on a Daudi Burkitt's cell line and compare with the natural wild-type IFNα-2 protein.

One example of determination of functionality is given hereinafter in the experimental part.

The invention is also directed to the use of polynucleotides and of polypeptides in conformity with the invention as well as of therapeutic molecules obtained and/or identified starting from these polynucleotides and polypeptides, notably for the prevention and the treatment of certain human disorders and/or diseases.

Figure 1 represents a model of the encoded protein according to the invention comprising the SNP M148I and the natural wild-type IFNα-2 protein.

Figure 2 represents a close up of the model of the right part of each one of the proteins represented in Figure 1.

The darker ribbon of Figure 1 represents the structure of the natural wild-type IFNα-2 protein. The lighter ribbon of Figure 1 represents the structure of the M148I mutated IFNα-2 protein.

Figure 3 represents the results of the test for measuring the antiproliferative effect of a polypeptide of the invention and a polypeptide encoded by the reference wild-type gene of human IFNα-2, on the Daudi Burkitt's cell line.

In this figure, the abscissas correspond to the logarithm of the protein concentration in picomoles (pM) and the ordinates correspond to the percentage of cell proliferation.

The antiproliferative effect of the wild-type IFNα-2 is represented by triangles and the antiproliferative effect of the mutated IFNα-2 is represented by circles.

Figure 4 represents the survival rate of mice previously infected by VSV virus and treated with M148I mutated IFNα-2, in comparison to those treated with wild-type IFNα-2, or those that have not been treated.

In this figure, the abscissas correspond to the relative survival rate of VSV infected mice and the ordinates correspond to the time of survival (days). The black triangles represent the data for VSV infected mice treated with M148I mutated IFNα-2, the black squares represent the data for VSV infected mice treated with wild-type IFNα-2, and the open triangles represent the data for VSV infected mice that have not been treated.

## DETAILED DESCRIPTION OF THE INVENTION

### Definitions

By "nucleotide sequence of the reference wild-type gene" is understood the nucleotide sequence SEQ ID N° 1 of the human gene.

This sequence is accessible in GenBank under Accession number J00207, V11834 and described in Olopade OI., Bohlander Sk. "Mapping of the shortest region of overlap of deletions of the short arm of chromosome 9 associated with human neoplasia." Genomics. 1992 Oct;14(2):437-43.

By "natural wild-type IFNα-2 protein" is understood the mature protein encoded by the nucleotide sequence of the reference wild-type IFNα-2 gene. The natural wild-type immature IFNα-2 protein corresponds to the peptide sequence SEQ ID N°2.

By "polynucleotide" is understood a polyribonucleotide or a polydeoxyribonucleotide that can be a modified or non-modified DNA or an RNA.

The term polynucleotide includes, for example, a single strand or double strand DNA, a DNA composed of a mixture of one or several single strand region(s) and of one or several double strand region(s), a single strand or double strand RNA and an RNA composed of a mixture of one or several single strand region(s) and of one or several double strand region(s). The term polynucleotide can also include an RNA and/or a DNA including one or several triple strand regions. By polynucleotide is equally understood the DNAs and RNAs containing one or several bases modified in such a fashion as to have a skeleton modified for reasons of stability or for other reasons. By modified base is understood, for example, the unusual bases such as inosine.

By "polypeptide" is understood a peptide, an oligopeptide, an oligomer or a protein comprising at least two amino acids joined to each other by a normal or modified peptide bond, such as in the cases of the isosteric peptides, for example.

A polypeptide according to the invention can be composed of amino acids other than the 20 amino acids defined by the genetic codeand can equally be composed of amino acids modified by natural processes, such as post translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can also be branched following an ubiquitination or be cyclic with or without branching. This type of modification can be the result of natural or synthetic post-translational processes that are well known to a person skilled in the art.

Polypeptide modifications may include, for example, acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, cysteine formation, pyroglutamate formation, formylation, gamma-carboxylation, glycosylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature: PROTEINS-STRUCTURE AND MOLECULAR PROPERTIES, 2$^{nd}$ Ed., T. E. Creighton, New York, 1993, POST-TRANSLATIONAL COVALENT MODIFICATION OF PROTEINS, B. C. Johnson, Ed., Academic Press, New York, 1983, Seifter et al. "Analysis for protein modifications and nonprotein cofactors", Meth. Enzymol. (1990) 182: 626-646, and Rattan et al. "Protein Synthesis: Post-translational Modifications and Aging", Ann NY Acad Sci (1992) 663: 48-62.

By "isolated polynucleotide" or "isolated polypeptide" is understood a polynucleotide or a polypeptide such as previously defined which is isolated from the human body or otherwise produced by a technical process.

By "identity" is understood the measurement of nucleotide or polypeptide sequence identity.

Identity is a term well known to a person skilled in the art and well described in the literature. See COMPUTATIONAL MOLECULAR BIOLOGY, Lesk, A.M., Ed., Oxford University Press, New York, 1998; BIOCOMPUTING INFORMATICS AND GENOME PROJECT, Smith, D.W., Ed., Academic Press, New York, 1993; COMPUTER ANALYSIS OF SEQUENCE DATA, PART I, Griffin, A.M. and Griffin H.G., Ed, Humana Press, New Jersey, 1994; and SEQUENCE ANALYSIS IN MOLECULAR BIOLOGY, von Heinje, G., Academic Press, 1987.

The methods commonly employed to determine the identity and the similarity between two sequences are equally well described in the literature. See GUIDE TO HUGE COMPUTER, Martin J. Bishop, Ed, Academic Press, San Diego, 1994, and Carillo H. and Lipton D., Siam J Applied Math (1988) 48: 1073.

A polynucleotide having, for example, an identity of at least 95 % with the nucleotide sequence SEQ ID N° 1 is a polynucleotide which contains at most 5 points of mutation over 100 nucleotides, compared to said sequence.

These points of mutation can be one (or several) substitution(s), addition(s) and/or deletion(s) of one (or several) nucleotide(s).

In the same way, a polypeptide having, for example, an identity of at least 95 % with the amino acid sequence SEQ ID N° 2 is a polypeptide that contains at most 5 points of mutation over 100 amino acids, compared to said sequence.

These points of mutation can be one (or several) substitution(s), addition(s) and/or deletion(s) of one (or several) amino acid(s).

The polynucleotides and the polypeptides according to the invention which are not totally identical with, respectively, the nucleotide sequence SEQ ID N°1 or the amino acid sequence SEQ ID N°2, containing at least one of the SNPs of the invention, are considered as variants of these sequences.

In general, polynucleotides according to the invention possess the same or practically the same biological activity as the nucleotide sequence SEQ ID N°1 comprising at least one of the SNPs of the invention.

In similar fashion, polypeptides according to the invention generally possess the same or practically the same biological activity as the amino acid sequence SEQ ID N°2 comprising at least one of the coding SNPs of the invention.

A variant, according to the invention, can be obtained, for example, by site-directed mutagenesis or by direct synthesis.

By "SNP" is understood any natural variation of a base in a nucleotide sequence. A SNP, in a nucleotide sequence, can be coding, silent or non-coding.

A coding SNP is a polymorphism in the coding sequence that involves a modification of one or more amino acids in the sequence of amino acids encoded by this nucleotide sequence. In this case, the term SNP applies equally, by extension, to a mutation in an amino acid sequence.
A silent SNP is a polymorphism in the coding sequence of a nucleotide sequence that does not involve a modification of any amino acid in the amino acid sequence encoded by this nucleotide sequence.
A non-coding SNP is a polymorphism in the non-coding sequence of a nucleotide sequence. This polymorphism can notably be found in an intron, a splicing zone, a transcription promoter or an enhancer site sequence.
By "functional SNP" is understood a SNP, such as previously defined, which is included in a nucleotide sequence or an amino acid sequence, and has some functionality.
By "functionality" is understood the biological activity of a polypeptide or of a polynucleotide.
The functionality of a polypeptide or of a polynucleotide according to the invention can consist in a conservation, an augmentation, a reduction or a suppression of the biological activity of the polypeptide encoded by the nucleotide sequence of the reference wild-type gene or of this latter nucleotide sequence.
The functionality of a polypeptide or of a polynucleotide according to the invention can equally consist of a change in the nature of the biological activity of the polypeptide encoded by the nucleotide sequence of the reference wild-type gene or of this latter nucleotide sequence.
The biological activity can, notably, be linked to the affinity or to the absence of affinity of a polypeptide according to the invention with a receptor.

<u>Polynucleotide</u>

The present invention is directed to an isolated polynucleotide comprising:

a) a nucleotide sequence having at least 80 % identity, preferably at least 90 % identity, more preferably at least 95 % identity and still more preferably at least 99 % identity with the sequence SEQ ID N° 1 or its coding sequence (nucleotide 511 to nucleotide 1077), it being understood that this nucleotide sequence comprises at least one of the following coding SNPs : c527a or g1023a, or
b) a nucleotide sequence complementary to a nucleotide sequence under a).

The present invention relates equally to an isolated polynucleotide comprising:

a) the nucleotide sequence SEQ ID N° 1 or its coding sequence, it being understood that each of these sequences comprises at least one of the following coding SNPs: c527a or g1023a, or
b) a nucleotide sequence complementary to a nucleotide sequence under a).

Preferably, the polynucleotide of the invention consists of the sequence SEQ ID N° 1 or its coding sequence, it being understood that each of these sequences comprises at least one of the following coding SNPs: c527a, g1023a.
According to the invention, the polynucleotide previously defined comprises a single coding SNP selected from the group consisting of: c527a and g1023a.
A polynucleotide such as previously defined can also include at least one of the following non-coding and silent SNPs: 96-100del(aattt), t110c, 139-144del(acttta), t338a, t363c, c427t, c1047t.
The present invention is also directed to an isolated polynucleotide comprising or consisting of:

a) the nucleotide sequence SEQ ID N° 1 or if necessary its coding sequence, such that each of these sequences comprises at least one of the following non coding or silent SNPs: 96-100del(aattt), t110c, 139-144del(acttta), t338a, t363c, c427t or c1047t; or
b) a nucleotide sequence complementary to a nucleotide sequence under a).

It is understood that only the silent SNP c1047t is located in the coding sequence of the nucleotide sequence SEQ ID N° 1.
The present invention also concerns an isolated polynucleotide consisting of a part of:

a) a nucleotide sequence SEQ ID N° 1 or if necessary its coding sequence, such that each one of these sequences contains at least one of the following SNPs: 96-100del(aattt), t110c, 139-144del(acttta), t338a, t363c, c427t, c527a, g1023a, c1047t ; or
b) a nucleotide sequence complementary to a nucleotide sequence under a);
said isolated polynucleotide being composed of at least 10 nucleotides.

Preferably, the isolated polynucleotide as defined above is composed of 10 to 40 nucleotides.

The present invention is also directed to an isolated polynucleotide coding for a polypeptide comprising:

a) the amino acid sequence SEQ ID N° 2, or

b) the amino acid sequence comprising the amino acids included between positions 24 and 188 of the sequence of amino acids SEQ ID N° 2;

it being understood that each of the amino acid sequences under a) and b) comprises at least one of the following coding SNPs: A6D, M171I.

It is understood, in the sense of the present invention, that the numbering corresponding to the positioning of the A6D, M171I SNPs is relative to the numbering of the amino acid sequence SEQ ID N°2.

In a preferred embodiment of the invention, the previously defined polypeptide comprises a single coding SNP such as defined above.

Preferably a polynucleotide according to the invention is composed of a DNA or RNA molecule.

A polynucleotide according to the invention can be obtained by standard DNA or RNA synthetic methods.

A polynucleotide according to the invention can equally be obtained by site-directed mutagenesis starting from the nucleotide sequence of the IFNα-2 gene by modifying the wild-type nucleotide by the mutated nucleotide for each SNP on the nucleotide sequence SEQ ID N° 1.

For example, a polynucleotide according to the invention, comprising a SNP g1023a can be obtained by site-directed mutagenesis starting from the nucleotide sequence of the IFNα-2 gene by changing the guanosine (g) at position 1023 in the nucleotide sequence SEQ ID N°1 to adenosine (a).

The processes of site-directed mutagenesis that can be implemented in this way are well known to a person skilled in the art, see the publication of TA Kunkel in 1985 in "Proc. Natl. Acad. Sci. USA" 82:488.

An isolated polynucleotide can equally include, for example, nucleotide sequences coding for pre-, pro- or pre-pro-protein amino acid sequences or marker amino acid sequences, such as hexa-histidine peptide.

A polynucleotide of the invention can equally be associated with nucleotide sequences coding for other proteins or protein fragments in order to obtain fusion proteins or other purification products.

A polynucleotide according to the invention can equally include nucleotide sequences such as the 5' and/or 3' non-coding sequences, such as, for example, transcribed or non-transcribed sequences, translated or non-translated sequences, splicing signal sequences, polyadenylated sequences, ribosome binding sequences or even sequences which stabilize mRNA.

A nucleotide sequence complementary to the nucleotide or polynucleotide sequence is defined as one that can hybridize with this nucleotide sequence, under stringent hybridization conditions.

By "stringent hybridization conditions" is generally but not necessarily understood the chemical conditions that permit hybridization only when the nucleotide sequences have an identity of at least 80 %, preferably greater than or equal to 90 %, still more preferably greater than or equal to 95 % and most preferably greater than or equal to 97 %.

The stringent conditions can be obtained according to methods well known to a person skilled in the art and, for example, by an incubation of the polynucleotides, at 42° C, in a solution comprising 50 % formamide, 5xSSC (150 mM of NaCl, 15 mM of trisodium citrate), 50 mM of sodium phosphate (pH = 7.6), 5x Denhardt Solution, 10 % dextran sulfate and 20 μg denatured salmon sperm DNA, followed by washing the filters at 0.1x SSC, at 65° C.

Within the scope of the invention, when the stringent hybridization conditions only permit hybridization of the nucleotide sequences having an identity equal to 100 %, the nucleotide sequence is considered to be strictly complementary to the nucleotide sequence such as described under a).

It is understood within the meaning of the present invention that the nucleotide sequence complementary to a nucleotide sequence comprises at least one anti-sense SNP according to the invention.

Thus, for example, if the nucleotide sequence comprises the SNP g1023a, its complementary nucleotide sequence comprises the thymine nucleotide (t) at the equivalent of position 1023.

Identification, hybridization and/or amplification of a polynucleotide comprising a SNP

The present invention is also directed to the use of all or part of:

a) a polynucleotide having 80 to 100 % identity with the nucleotide sequence SEQ ID N°1, and/or

b) a polynucleotide according to the invention comprising at least one SNP;

in order to identify, hybridize and/or amplify all or part of a polynucleotide having 80 to 100 % identity with the nucleotide sequence SEQ ID N° 1 or if necessary its coding sequence (nucleotide 511 to nucleotide 1077), it being understood that each one of these sequences contains at least one of the following SNPs: 96-100del(aattt), t110c, 139-144del(acttta), t338a, t363c, c427t, c527a, g1023a, c1047t.

Genotyping and determination of the frequency of a SNP

The present invention equally has for its object the use of all or part of:

a) a polynucleotide having 80 to 100 % identity (preferably at least 90 % identity, more preferably 95 % identity and particularly 100 % identity) with the nucleotide sequence SEQ ID N°1, and/or
b) a polynucleotide according to the invention comprising at least one SNP;
for the genotyping of all or part of a polynucleotide having 80 to 100 % identity (preferably at least 90 % identity, more preferably 95 % identity and particularly 100 % identity) with the nucleotide sequence SEQ ID N° 1 or if necessary its coding sequence (of the nucleotide 511 to the nucleotide 1077 ), it being understood that each one of these sequences comprises at least one of the following SNPs: 96-100del(aattt), t110c, 139-144del(acttta), t338a, t363c, c427t, c527a, g1023a, c1047t.

According to the invention, the genotyping may be carried out on an individual or a population of individuals, most preferably in a population of individuals. A genotype consists of the determination of the alleles present at one or more specific loci.
Within the meaning of the invention, genotyping is defined as a process for the determination of the genotype of an individual or of a population of individuals. Genotype consists of the alleles present at one or more specific loci.
By "population of individuals" is understood a group of determined individuals selected in random or non-random fashion. These individuals can be humans, animals, microorganisms or plants. Usually, the group of individuals comprises at least 10 persons, preferably from 100 to 300 persons.
The individuals can be selected according to their ethnicity or according to their phenotype, notably those who are affected by the following disorders and/or diseases:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,
- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,
- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

Multiple technologies exist which can be implemented in order to genotype SNPs (see for example Kwok Pharmacogenomics, 2000, vol 1, 95-100. "High-throughput genotyping assay approaches"). These technologies are based on one of the four following principles: allele specific oligonucleotide hybridization, oligonucleotide elongation by dideoxynucleotides optionally in the presence of deoxynucleotides, ligation of allele specific oligonucleotides or cleavage of allele specific oligonucleotides. Each one of these technologies can be coupled to a detection system such as measurement of direct or polarized fluorescence, or mass spectrometry.
Genotyping can notably be carried out by minisequencing with hot ddNTPs (2 different ddNTPs labeled by different fluorophores) and cold ddNTPs (2 different non labeled ddNTPs), in connection with a polarized fluorescence scanner. The minisequencing protocol with reading of polarized fluorescence (FP-TDI Technology or Fluorescence Polarization Template-direct Dye-Terminator Incorporation) is well known to a person skilled in the art.
FP-TDI can be carried out on a product obtained after amplification by polymerase chain reaction (PCR) of the DNA of each individual. This PCR product is selected to cover the polynucleotide genic region containing the studied SNP. After the last step in the PCR thermocycler, the plate is then placed on a polarized fluorescence scanner for a reading of the labeled bases by using fluorophore specific excitation and emission filters. The intensity values of the labeled bases are reported on a graph.

For the PCR amplification, in the case of a SNP of the invention, the sense and antisense primers, respectively, can easily be selected by a person skilled in the art according to the position of the SNPs of the invention in order to amplify a region of interest containing the SNP or SNPs.

For example, a first set of sense and antisense nucleotide sequences for the PCR amplification can be:

Sense primer: GCCTCTTATGTACCCACAAA

Antisense primer: CACCAGTAAAGCAAAGGTCA

These nucleotide sequences permit amplification of a fragment having a length of 535 nucleotides, from nucleotide 3 to nucleotide 537 in the nucleotide sequence SEQ ID N° 1.

For example, a second set of sense and antisense nucleotide sequences for the PCR amplification can be:

Sense primer: CACCCATTTCAACCAGTCTA

Antisense primer: AGCTGGCATACGAATCAAT

These nucleotide sequences permit amplification of a fragment having a length of 655 nucleotides, from nucleotide 470 to nucleotide 1124 in the nucleotide sequence SEQ ID N° 1.

A statistical analysis of the frequency of each allele (allelic frequency) encoded by the gene comprising the SNP in the population of individuals is then achieved, which permits determination of the importance of their impact and their distribution in the different sub-groups including the diverse ethnic groups that constitute this population of individuals.

The genotyping data are analyzed in order to estimate the distribution frequency of the different alleles observed in the studied populations. The calculations of the allelic frequencies can be carried out with the help of software such as SAS-suite® (SAS) or SPLUS® (MathSoft). The comparison of the allelic distributions of a SNP of the invention across different ethnic groups of the population of individuals can be carried out by means of software such as ARLEQUIN® and SAS-suite®.

SNPs of the invention as genetic markers

Whereas SNPs modifying functional sequences of genes (e.g. promoter, splicing sites, coding region) are likely to be directly related to disease susceptibility or resistance, all SNPs (functional or not) may provide valuable markers for the identification of one or several genes involved in these disease states and, consequently, may be indirectly related to these disease states (See Cargill et al. (1999). Nature Genetics 22:231-238; Riley et al. (2000). Pharmacogenomics 1:39-47; Roberts L. (2000). Science 287: 1898-1899).

Thus, the present invention also concerns a databank comprising at least one of the following SNPs: 96-100del (aattt), t110c, 139-144del(acttta), t338a, t363c, c427t, c527a, g1023a, c1047t, in a polynucleotide of the IFNα-2 gene.

It is well understood that said SNPs are numbered in accordance with the nucleotide sequence SEQ ID N°1.

This databank may be analyzed for determining statistically relevant associations between: at least one of the following SNPs: 96-100del(aattt), t110c, 139-144del(acttta), t338a, t363c, c427t, c527a, g1023a, c1047t, in a polynucleotide of the IFNα-2 gene, and a disease or a resistance to a disease, comprising

a) genotyping a group of individuals,
b) determining the distribution of said disease or resistance to disease within said group of individuals,
c) comparing the genotype data with the distribution of said disease or resistance to disease, and
d) analyzing said comparison for statistically relevant associations.

The present invention also concerns the use of at least one of the following SNPs: 96-100del(aattt), t110c, 139-144del(acttta), t338a, t363c, c427t, c527a, g1023a, c1047t, in a polynucleotide of the IFNα-2 gene, for developing diagnostic/prognostic kits for a disease or a resistance to a disease.

A SNP of the invention such as defined above may be directly or indirectly associated to a disease or a resistance to a disease.

Preferably, these diseases may be those which are defined as mentioned above.

Expression vector and host cell

The present invention is also directed to a recombinant vector comprising at least one polynucleotide according to the invention.
Numerous expression systems can be used, including, for example, chromosomes, episomes, derived viruses. More particularly, the recombinant vectors used can be derived from bacterial plasmids, transposons, yeast episome, insertion elements, yeast chromosome elements, viruses such as baculovirus, papilloma viruses such as SV40, vaccinia viruses, adenoviruses, fox pox viruses, pseudorabies viruses, retroviruses.
These recombinant vectors can equally be cosmid or phagemid derivatives. The nucleotide sequence can be inserted in the recombinant expression vector by methods well known to a person skilled in the art such as, for example, those that are described in MOLECULAR CLONING, A LABORATORY MANUAL (supra) Sambrook et al.
The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.
Thus, for example, an appropriate secretion signal can be integrated in the recombinant vector so that the polypeptide, encoded by the polynucleotide of the invention, will be directed towards the lumen of the endoplasmic reticulum, towards the periplasmic space, on the membrane or towards the extracellular environment.
The present invention is also directed to a host cell comprising a recombinant vector according to the invention.
The introduction of the recombinant vector in a host cell can be carried out according to methods that are well known to a person skilled in the art such as those described in BASIC METHODS IN MOLECULAR BIOLOGY, Davis et al., 1986 and MOLECULAR CLONING: A LABORATORY MANUAL, 2$^{nd}$ Ed., Cold Spring Harbor Laboratory Press, Cold Spring Harbor, N.Y., 1989, such as transfection by calcium phosphate, transfection by DEAE dextran, transfection, microinjection, transfection by cationic lipids, electroporation, transduction or infection.
The host cell can be, for example, bacterial cells such as cells of streptococci, staphylococci, *E. coli* or *Bacillus subtilis,* cells of fungi such as yeast cells and cells of *Aspergillus, Streptomyces,* insect cells such as cells of *Drosophilia* S2 and of *Spodoptera* Sf9, animal cells, such as CHO, COS, HeLa, C127, BHK, HEK 293 cells and human cells of the subject to treat or even plant cells.
The host cells can be used, for example, to express a polypeptide of the invention or as an active product in pharmaceutical compositions, as will be seen hereinafter.

Polypeptides

The present invention is also directed to an isolated polypeptide comprising an amino acid sequence having at least 80 % identity, preferably at least 90 % identity, more preferably at least 95 % identity and still more preferably at least 99 % identity with:

a) the amino acid sequence SEQ ID N° 2, or with
b) the amino acid sequence comprising the amino acids included between positions 24 and 188 of the amino acid sequence SEQ ID N° 2;
it being understood that each of the amino acid sequences under a) and b) contains at least one of the following coding SNPs: A6D, M171I.

The polypeptide of the invention can equally comprise:

a) the amino acid sequence SEQ ID N° 2, or
b) the amino acid sequence containing the amino acids included between positions 24 and 188 of the amino acid sequence SEQ ID N° 2;
it being understood that each of the amino acid sequences under a) and b) contains at least one of the following coding SNPs: A6D, M171I.

The polypeptide of the invention can more particularly consist of:

a) the amino acid sequence SEQ ID N° 2, or
b) the amino acid sequence containing the amino acids included between positions 24 and 188 of the amino acid sequence SEQ ID N° 2;

it being understood that each one of the amino acid sequences under a) and b) contains at least one of the following coding SNPs: A6D, M171I.

Preferably, a polypeptide according to the invention contains a single coding SNP selected from the group consisting of: A6D and M171I.
The present invention equally has for its object a process for the preparation of the above-described polypeptide, in which a previously defined host cell is cultivated in a culture medium and said polypeptide is isolated from the culture medium.
The polypeptide can be purified starting from the host cells' culture medium, according to methods well known to a person skilled in the art such as precipitation with chaotropic agents such as salts, in particular ammonium sulfate, ethanol, acetone or trichloroacetic acid, acid extraction; ion exchange chromatography; phosphocellulose chromatography; hydrophobic interaction chromatography; affinity chromatography; hydroxyapatite chromatography or exclusion chromatographies.
By "culture medium" is understood the medium in which the polypeptide of the invention is isolated or purified. This medium can be composed of the extracellular medium and/or the cellular lysate. Techniques well known to a person skilled in the art also permit the latter to produce an active conformation to the polypeptide, if the conformation of said polypeptide was altered during the isolation or the purification.

Antibodies

The present invention also concerns a process for obtaining an immunospecific antibody.
By "antibody" is understood the monoclonal, polyclonal, chimeric, simple chain and/or humanized antibodies as well as the Fab fragments, including Fab or immunoglobulin expression library products.
An immunospecific antibody can be obtained by immunization of an animal with a polypeptide according to the invention.
The invention also relates to an immunospecific antibody for a polypeptide according to the invention, such as defined previously.
A polypeptide according to the invention, one of its fragments, an analog, one of its variants or a cell expressing this polypeptide can also be used to produce immunospecific antibodies.
The term "immunospecific" means that the antibody possesses a better affinity for the polypeptide of the invention than for other polypeptides known in the prior art.
The immunospecific antibodies can be obtained by administration of a polypeptide of the invention, of one of its fragments, of an analog or of an epitopic fragment or of a cell expressing this polynucleotide in a mammal, preferably non human, according to methods well known to a person skilled in the art.
For the preparation of monoclonal antibodies, typical methods for antibody production can be used, starting from cell lines, such as the hybridoma technique (Kohler et al., Nature (1975) 256: 495-497), the trioma technique, the human B cell hybridoma technique (Kozbor et al., Immunology Today (1983) 4: 72) and the EBV hybridoma technique (Cole et al., MONOCLONAL ANTIBODIES AND CANCER THERAPY, pp. 77-96, Alan R. Liss, 1985).
The techniques of single chain antibody production such as described, for example, in US Patent N° 4,946, 778 can also be used.
Transgenic animals such as mice, for example, can also be used to produce humanized antibodies.

Agents interacting with the polypeptide of the invention

The present invention equally has for its object a process for the identification of an agent activating or inhibiting a polypeptide according to the invention, comprising:

a) the preparation of a recombinant vector comprising a polynucleotide according to the invention containing at least one coding SNP,
b) the preparation of host cells comprising a recombinant vector according to a),
c) the contacting of host cells according to b) with an agent to be tested, and
d) the determination of the activating or inhibiting effect generated by the agent to test.

A polypeptide according to the invention can also be employed for screening compounds that interact with it.
These compounds can be activating (agonists) or inhibiting (antagonists) agents of intrinsic activity of a polypeptide according to the invention. These compounds can equally be ligands or substrates of a polypeptide of the invention. See Coligan et al., Current Protocols in Immunology 1 (2), Chapter 5 (1991).
In general, in order to implement such a process, it is first desirable to produce appropriate host cells that express a polypeptide according to the invention. Such cells can be, for example, cells of mammals, yeasts, insects such as *Drosophilia* or bacteria such as *E. coli.*

These cells or membrane extracts of these cells are then put in the presence of compounds to be tested.

The binding capacity of the compounds to be tested with the polypeptide of the invention can then be observed, as well as the inhibition or the activation of the functional response.

Step d) of the above process can be implemented by using an agent to be tested that is directly or indirectly labeled. It can also include a competition test, by using a labeled or non-labeled agent and a labeled competitor agent.

It can also be determined if an agent to be tested generates an activation or inhibition signal on cells expressing the polypeptide of the invention by using detection means appropriately chosen according to the signal to be detected.

Such activating or inhibiting agents can be polynucleotides, and in certain cases oligonucleotides or polypeptides, such as proteins or antibodies, for example.

The present invention also concerns a process for the identification of an agent activated or inhibited by a polypeptide according to the invention, comprising:

a) the preparation of a recombinant vector comprising a polynucleotide according to the invention containing at least one coding SNP,
b) the preparation of host cells comprising a recombinant vector according to a),
c) placing host cells according to b) in the presence of an agent to be tested, and
d) the determination of the activating or inhibiting effect generated by the polypeptide on the agent to be tested.

An agent activated or inhibited by the polypeptide of the invention is an agent that responds, respectively, by an activation or an inhibition in the presence of this polypeptide. The agents, activated or inhibited directly or indirectly by the polypeptide of the invention, can consist of polypeptides such as, for example, membrane-bound or nuclear receptors, kinases and more preferably tyrosine kinases, transcription factor or polynucleotides.

Detection of diseases

The present invention also has for an object a process for analyzing the biological characteristics of a polynucleotide according to the invention and/or of a polypeptide according to the invention in a subject, comprising at least one of the following:

a) Determining the presence or the absence of a polynucleotide according to the invention in the genome of a subject,
b) Determining the level of expression of a polynucleotide according to the invention in a subject,
c) Determining the presence or the absence of a polypeptide according to the invention in a subject,
d) Determining the concentration of a polypeptide according to the invention in a subject, and/or
e) Determining the functionality of a polypeptide according to the invention in a subject.

These biological characteristics may be analyzed in a subject or in a sample from a subject.

These biological characteristics may permit to carry out a genetic diagnosis and to determine whether a subject is affected or at risk of being affected or, to the contrary, presents a partial resistance to the development of a disease, an indisposition or a disorder linked to the presence of a polynucleotide according to the invention and/or a polypeptide according to the invention.

These diseases can be disorders and/or human diseases, such as:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,
- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,

- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

This process also permits a genetic diagnosis of a disease or of a resistance to a disease linked to the presence, in a subject, of the mutant allele encoded by a SNP according to the invention.
Preferably, in step a), the presence or absence of a polynucleotide, containing at least one coding SNP such as previously defined, will be detected.
The detection of the polynucleotide may be carried out starting from biological samples from the subject to be studied, such as cells, blood, urine, saliva, or starting from a biopsy or an autopsy of the subject to be studied. The genomic DNA may be used for the detection directly or after a PCR amplification, for example. RNA or cDNA can equally be used in a similar fashion.
It is then possible to compare the nucleotide sequence of a polynucleotide according to the invention with the nucleotide sequence detected in the genome of the subject.
The comparison of the nucleotide sequences can be carried out by sequencing, by DNA hybridization methods, by mobility difference of the DNA fragments on an electrophoresis gel with or without denaturing agents or by melting temperature difference. See Myers et al., Science (1985) 230: 1242. Such modifications in the structure of the nucleotide sequence at a precise point can equally be revealed by nuclease protection tests, such as RNase and the S1 nuclease or also by chemical cleaving agents. See Cotton et al., Proc. Nat. Acad. Sci. USA (1985) 85: 4397-4401. Oligonucleotide probes comprising a polynucleotide fragment of the invention can equally be used to conduct the screening.
Many methods well known to a person skilled in the art can be used to determine the expression of a polynucleotide of the invention and to identify the genetic variability of this polynucleotide (See Chee et al., Science (1996), Vol 274, pp 610-613).
In step b), the level of expression of the polynucleotide may be measured by quantifying the level of RNA encoded by this polynucleotide (and coding for a polypeptide) according to methods well known to a person skilled in the art such as, for example, by PCR, RT-PCR, RNase protection, Northern blot, and other hybridization methods.
In step c) and d) the presence or the absence as well as the concentration of a polypeptide according to the invention in a subject or a sample form a subject may be carried out by well known methods such as, for example, by radioimmunoassay, competitive binding tests, Western blot and ELISA tests.
Consecutively to step d), the determined concentration of the polypeptide according to the invention can be compared with the natural wild-type protein concentration usually found in a subject.
A person skilled in the art can identify the threshold above or below which appears the sensitivity or, to the contrary, the resistance to the disease, the indisposition or the disorder evoked above, with the help of prior art publications or by conventional tests or assays, such as those that are previously mentioned.
In step e), the determination of the functionality of a polypeptide according to the invention may be carried out by methods well known to a person skilled in the art as, for example, by in vitro tests such as above mentioned or by an use of host cells expressing said polypeptide.

Medicaments and treatments of diseases

The polypeptides of the invention possess very interesting pharmacological properties. In particular, they can bind to the human IFN$\alpha$-2 receptor. These properties are in accordance with the use of the polypeptides of the invention for therapeutic treatment of human body, i.e. as a medicament or therapeutic composition.
Thus, the present invention is also directed to a medicament containing, by way of active agent, a polypeptide according to the invention.
The invention also relates to the use of a polypeptide according to the invention, for the manufacture of a medicament intended for the prevention or the treatment of different human disorders and/or diseases, such as:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,

- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,
- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

Certain of the compounds permitting to obtain the polypeptide according to the invention as well as the compounds obtained or identified by or from this polypeptide can likewise be used for the therapeutic treatment of the human body, i.e. as a medicament.

This is why the present invention also has for an object a medicament containing, by way of active agent, a polynucleotide according to the invention containing at least one previously defined coding SNP, a previously defined recombinant vector, a previously defined host cell, and/or a previously defined antibody.

The invention also relates to the use of a polynucleotide according to the invention containing at least one previously defined coding SNP, a previously defined recombinant vector, a previously defined host cell, and/or a previously defined antibody, for the manufacture of a medicament intended for the prevention or the treatment of different human disorders and/or diseases, such as:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,
- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,
- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

The dosage of a polypeptide and of the other compounds of the invention, useful as active agent, depends on the choice of the compound, the therapeutic indication, the mode of administration, the nature of the formulation, the nature of the subject and the judgment of the doctor.

When it is used as active agent, a polypeptide according to the invention is generally administered at doses ranging between 1 and 100 μg/kg of the subject.

The invention also has as an object a pharmaceutical composition that contains, as active agent, at least one above-mentioned compound such as a polypeptide according to the invention, a polynucleotide according to the invention containing at least one previously defined SNP, a previously defined recombinant vector, a previously defined host cell, and/or a previously defined antibody, as well as a pharmaceutically acceptable excipient.

In these pharmaceutical compositions, the active agent is advantageously present at physiologically effective doses.

These pharmaceutical compositions can be, for example, solids or liquids and be present in pharmaceutical forms currently used in human medicine such as, for example, simple or coated tablets, gelcaps, granules, caramels, suppositories and preferably injectable preparations and powders for injectables. These pharmaceutical forms can be prepared according to usual methods.

The active agent(s) can be incorporated into excipients usually employed in pharmaceutical compositions such as talc, Arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, preservatives.

The active agent(s) according to the invention can be employed alone or in combination with other compounds such as therapeutic compounds such as other interferons-$\alpha$ or other cytokines such as interleukine, for example.

The different formulations of the pharmaceutical compositions are adapted according to the mode of administration.

The pharmaceutical compositions can be administered by different routes of administration known to a person skilled in the art.

The invention equally has for an object a diagnostic composition that contains, as active agent, at least one above-mentioned compound such as a polypeptide according to the invention, all or part of a polynucleotide according to the invention, a previously defined recombinant vector, a previously defined host cell, and/or a previously defined antibody, as well as a suitable pharmaceutically acceptable excipient.

This diagnostic composition may contain, for example, an appropriate excipient like those generally used in the diagnostic composition such as buffers and preservatives.

The present invention equally has as an object the use:

a) of a therapeutically effective quantity of a polypeptide according to the invention, and/or
b) of a polynucleotide according to the invention, and/or
c) of a host cell from the subject to be treated, previously defined,
to prepare a medicament intended to increase the expression or the activity, in a subject, of a polypeptide according to the invention.

Thus, to treat a subject who needs an increase in the expression or in the activity of a polypeptide of the invention, several methods are possible.

It is possible to administer to the subject a therapeutically effective quantity of a polypeptide of the invention with a pharmaceutically acceptable excipient.

It is likewise possible to increase the endogenous production of a polypeptide of the invention by administration to the subject of a polynucleotide according to the invention. For example, this polynucleotide can be inserted in a retroviral expression vector. Such a vector can be isolated starting from cells having been infected by a retroviral plasmid vector containing RNA encoding for the polypeptide of the invention, in such a fashion that the transduced cells produce infectious viral particles containing the gene of interest. See Gene Therapy and other Molecular Genetic-based Therapeutic Approaches, Chapter 20, in Human Molecular Genetics, Strachan and Read, BIOS Scientifics Publishers Ltd (1996).

In accordance with the invention, a polynucleotide containing at least one coding SNP such as previously defined will be preferably used.

It is equally possible to administer to the subject host cells belonging to him, these host cells having been preliminarily taken and modified so as to express the polypeptide of the invention, as previously described.

The present invention equally relates to the use:

a) of a therapeutically effective quantity of a previously defined immunospecific antibody, and/or
b) of a polynucleotide permitting inhibition of the expression of a polynucleotide according to the invention;
in order to prepare a medicament intended to reduce the expression or the activity, in a subject, of a polypeptide according to the invention.

Thus, it is possible to administer to the subject a therapeutically effective quantity of an inhibiting agent and/or of an antibody such as previously defined, possibly in combination, with a pharmaceutically acceptable excipient.

It is equally possible to reduce the endogenous production of a polypeptide of the invention by administration to the subject of a complementary polynucleotide according to the invention permitting inhibition of the expression of a polynucleotide of the invention.

Preferably, a complementary polynucleotide containing at least one coding SNP such as previously defined can be used.

The present invention concerns also the use of a IFN$\alpha$-2 protein for the preparation of a medicament for the prevention or the treatment of a patient having a disorder or a disease caused by an IFN$\alpha$-2 variant linked to the presence in the genome of said patient of a nucleotide sequence having at least 95% identity (preferably, 97% identity, more preferably 99% identity and particularly 100% identity) with the nucleotide sequence SEQ ID N° 1, provided that said nucleotide sequence comprises one of the following SNPs: 96-100del(aattt), t110c, 139-144del(acttta), t338a, t363c, c427t, c527a, g1023a, c1047t.

Preferably, said medicament is used for the prevention or the treatment of one of the diseases selected from the group consisting of:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,
- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,
- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

<u>Mimetic compounds of a IFNα-2 polypeptide comprising the SNP M171I</u>

The present invention also concerns a new compound having a biological activity substantially similar or lower in comparison to that of M171I IFNα2 gene product.

The M171I mutated IFNα2 gene product corresponds to a polypeptide of:

a) amino acid sequence ID SEQ N°2, or

b) amino acid sequence comprising the amino acids included between positions 24 and 188 of the amino acid sequence SEQ ID N°2;

provided that said amino acid sequences under a) and b) comprise the M171I SNP.

Said biological activity may be evaluated, for example, by measuring cellular antiproliferative activity on Daudi Burkitt's cell line or signal transduction assays on MCF7 cells using luciferase reporter gene as described below in the experimental part.

As mentioned in the experimental part, the M171I mutated IFNα-2 possesses a cellular antiproliferative activity on Daudi Burkitt's cell line which is lower than that of the natural wild-type IFNα-2.

As mentioned in the experimental part, the signal transduction activation in the breast carcinoma cell line MCF-7 measured in presence of the M171I mutated IFNα-2 is lower than that measured with the natural wild-type IFNα-2.

A new compound of the invention, such as previously defined, may possess a biological activity substantially similar to that of the M171I mutated IFNα-2, i.e. which is lower than that of the natural wild-type IFNα-2.

Said compound may also have a biological activity which is even lower than that of the M171I mutated IFNα-2.

Said compound may be a biochemical compound, such as a polypeptide or a peptide for example, or an organic chemical compound, such as a synthetic peptide-mimetic for example.

The present invention also provides a new compound having a cellular antiproliferative activity on Daudi Burkitt's cell line at least 15 times lower than that of the natural wild-type IFNα-2.

The present invention also provides a new compound having a signal transduction capacity on MCF7 cells at least 10 times lower than that of the natural wild-type IFNα-2.

The present invention also concerns the use of a polypeptide of the invention containing the M171I SNP, for the identification of a compound such as defined above.

The present invention also concerns a process for the identification of a compound of the invention, comprising the following steps:

a) Determining the biological activity, such as the cellular antiproliferative activity on Daudi Burkitt's cell line or the signal transduction capacity,
b) Comparing the activity determined in step a) of the compound to be tested, with the activity of the M171I mutated IFNα-2 gene product, and
c) Determining on the basis of the comparison carried out in step b) whether the compound to be tested has a substantially similar or lower activity compared to that of the M171I mutated IFNα2 gene product.

Preferably, the compound to be tested may be previously identified from synthetic peptide combinatorial libraries, high-throughput screening, or designed by computer-aided drug design so as to have the same three-dimensional structure and/or chemical effect as that of the M171I mutated IFNα2 gene product. The methods to identify and design compounds are well known by a person skilled in the art.

Publications referring to these methods may be, for example:

- Silverman R.B. (1992). "Organic Chemistry of Drug Design and Drug Action". Academic Press, 1st edition (January 15, 1992).
- Anderson S and Chiplin J. (2002). "Structural genomics; shaping the future of drug design? Drug Discov. Today. 7(2):105-107.
- Selick HE, Beresford AP, Tarbit MH. (2002). "The emerging importance of predictive ADME simulation in drug discovery". Drug Discov. Today. 7(2): 109-116.
- Burbidge R, Trotter M, Buxton B, Holden S. (2001). "Drug design by machine learning: support vector machines for pharmaceutical data analysis". Comput. Chem. 26(1): 5-14.
- Kauvar L.M. (1996). "Peptide mimetic drugs: a comment on progress and prospects" 14(6): 709.

The compounds of the invention may be used for the preparation of a medicament intended for the prevention or the treatment of one of the diseases selected from the group consisting of:

- cancers and tumors, such as carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,
- cardiovascular diseases,
- metabolic diseases, such as non-immune associated diseases comprising obesity,
- infectious diseases, such as viral infections comprising chronic hepatitis B and C and HIV/AIDS, and infectious pneumonias,
- diseases of the central nervous system, such as Alzheimer's disease, Parkinson's disease, multiple sclerosis, schizophrenia and depression,
- immunologically and auto-immunologically related diseases, such as the rejection of tissue or organ grafts, allergies, asthma, psoriasis and rheumatoid arthritis,
- healing of wounds,
- disorders connected with chemotherapy treatment,
- anemia in dialyzed patient,
- osteoporosis,
- gastrointestinal disorders, such as Crohn's disease and ulcerative colitis, and
- venereal diseases, such as genital warts.

<u>EXPERIMENTAL PART</u>

<u>Example 1: Modeling of a protein encoded by a polynucleotide of nucleotide sequence containing the g1023a SNP and of the protein encoded by the nucleotide sequence of the reference wild-type gene</u>

In a first step the threedimensional structure of IFNα-2 was constructed starting from that of human IFNα-2 whose structure is available in the PDB database (code 1ITF) using the software Modeler (MSI, San Diego, CA).

The mature polypeptide fragment was then modified in such a fashion as to reproduce the mutation M148I.

A thousand molecular minimization steps were conducted on this mutated fragment by using the programs AMBER and DISCOVER (MSI: Molecular Simulations Inc.).

Two molecular dynamic calculation runs were then carried out with the same program and the same force fields.

In each case, 50,000 steps were calculated at 300°K, terminated by 300 equilibration steps.
The result of this modeling is visualized on Figures 1 and 2.

Example 2: Prediction of the addressing of the protein encoded by a polynucleotide of nucleotide sequence containing the c527a SNP and of the protein encoded by the nucleotide sequence of the reference wild-type gene

The c527a polymorphism involves an A6D polymorphism at the level of the immature IFNα-2 protein. This position is located on the signal peptide comprising the first 23 amino acids of the immature protein. The signal peptide controls the addressing of the protein and the intracellular or extracellular compartment where the mature protein will be expressed.
PSORT is a software available in the public domain that analyzes the amino acid sequence of the immature protein and predicts in which compartment the mature protein will be localized (Nakai K and Kanehisa M (1992).
A knowledge base for predicting protein localization sites in eukaryotic cells. Genomics 14, 897-911).
PSORT predicts that the A6D polymorphism changes the IFNα-2 localization at the cellular level. Indeed, it predicts that the wild-type mature IFNα-2 protein is secreted, meaning an extracellular localization, whereas the D6 mutated IFNα-2 will be in the nuclear and/or the mitochondrial compartments.
Even if the A6D mutant of IFNα-2 is probably not affected in its structure and function in comparison to the wild-type protein, its activity will not be found where it normally would have been.

Example 3. Prediction of changes in IFNα-2 expression caused by the deletion 139-144del(acttta) in the polvnucleotide sequence SEQ ID N°1

The effects of the 139-144del(acttta) deletion on putative binding sites have been evaluated after a run in Match (Transfac). Although this deletion is not located in the regulatory region common to all interferons, it cancels three putative binding sites for transcription factors:

- the putative binding site for Msx-1 encompasses nucleotides 134 to 142 on the wild-type reference sequence ID SEQ N°1. The effect of this deletion on this binding site cannot be estimated properly since this transcription factor is not well known and its function has been proposed to be related to limb development but has not been clearly identified (Hwang et al. (1998). Am. J. Med. Genet. 75:419-423).
- the putative binding site for Oct-1 encompasses nucleotides 141 to 163 on the wild-type reference sequence ID SEQ N°1. Although this binding site is located at about 400 nucleotides upstream of the transcription initiation site, its deletion may have an important effect since the transcription factor that recognizes this site is involved in different mechanisms of transcription initiation in lymphocytes T and B (Ullman et al. (1991). Science. 254:558-562 ; Kemler et al. (1989). EMBO J. 8:2001-2008 ; Kamps et al. (1990). Mol Cell Biol. 10:5464-5472).
- the putative binding site for Hoxa3 encompasses nucleotides 143 to 151 on the wild-type reference sequence ID SEQ N°1. Hoxa3 is a transcription factor that is involved in the regulation of fetal thymic epithelial cells to promote thymocyte development (Su et al. (2001). Dev. Biol. 236:316-329).

As a result, the 139-144del(acttta) SNP may affect the normal regulation of IFNα-2 expression.

Example 4: Genotyping of the SNPs t110c, t338a, t363c, c427t, c527a and g1023a in a population of individuals

The genotyping of SNPs is based on the principle of the minisequencing wherein the product is detected by reading polarized fluorescence. The technique consists of a fluorescent minisequencing (FP-TDI Technology or Fluorescence Polarization Template-direct Dye-terminator Incorporation).
The minisequencing is performed on a product amplified by PCR from genomic DNA of each individual of the population. The PCR product is chosen in such a manner that it covers the genic region containing the SNP to be genotyped. After elimination of the PCR primers that have not been used and the dNTPs that have not been incorporated, the minisequencing is carried out.
The minisequencing consists of lengthening an oligonucleotide primer, placed just upstream of the site of the SNP, by using a polymerase enzyme and fluorolabeled dideoxynucleotides. The product resulting from this lengthening process is directly analyzed by reading polarized fluorescence.
All these steps, as well as the reading, are carried out in the same PCR plate.
Thus, the genotyping requires 5 steps:

1) Amplification by PCR
2) Purification of the PCR product by enzymatic digestion

3) Elongation of the oligonucleotide primer
4) Reading polarized fluorescence
5) Interpretation of the reading

The genotyping steps 1 and 2 are carried out in the same conditions for each of the SNPs t110c, t338a, t363c and c427t on the one hand and each of the SNPs c527a, and g1023a on the other hand.
The steps 3, 4 and 5 are specific to each one of these polymorphisms.
1) The PCR amplification of the nucleotide sequence of the IFNα-2 gene is carried out starting from genomic DNA from 268 individuals of ethnically diverse origins.
These genomic DNAs were provided by the Coriell Institute in the United States.
The 268 individuals are distributed as follows:

| Phylogenic Population | Specific Ethnic Population | Total | % |
|---|---|---|---|
| **African American** | African American | *50* | *100.0* |
| | ***Subtotal*** | ***50*** | ***18.7*** |
| **Amerind** | South American Andes | *10* | *66.7* |
| | South West American Indians | *5* | *33.3* |
| | ***Subtotal*** | ***15*** | ***5.6*** |
| **Caribbean** | Caribbean | *10* | *100.0* |
| | ***Subtotal*** | ***10*** | ***3.7*** |
| **European Caucasoid** | North American Caucasian | *79* | *79.8* |
| | Iberian | *10* | *10.1* |
| | Italian | *10* | *10.1* |
| | ***Subtotal*** | ***99*** | ***36.9*** |
| **Mexican** | Mexican | *10* | *100.0* |
| | ***Subtotal*** | ***10*** | ***3.7*** |
| **Northeast Asian** | Chinese | *10* | *50.0* |
| | Japanese | *10* | *50.0* |
| | ***Subtotal*** | ***20*** | ***7.5*** |
| **Non-European Caucasoid** | Greek | *8* | *21.6* |
| | Indo-Pakistani | *9* | *24.3* |
| | Middle-Eastern | *20* | *54.1* |
| | ***Subtotal*** | ***37*** | ***13.8*** |
| **Southeast Asian** | Pacific Islander | *7* | *41.2* |
| | South Asian | *10* | *58.8* |
| | ***Subtotal*** | ***17*** | ***6.3*** |
| **South American** | South American | *10* | *100.0* |
| | ***Subtotal*** | ***10*** | ***3.7*** |
| | ***Total*** | ***268*** | ***100*** |

The genomic DNA coming from each one of these individuals constitutes a sample.
For SNPs t110c, t338a, t363c and c427t, the PCR amplification is carried out starting from the following primers:

Sense primer: GCCTCTTATGTACCCACAAA

Antisense primer: CACCAGTAAAGCAAAGGTCA

These nucleotide sequences permit amplification of a fragment of a length of 535 nucleotides, nucleotide 3 to nucleotide 537, in the nucleotide sequence SEQ ID N° 1.

For SNPs c527a, and g1023a, the PCR amplification is carried out starting from the following primers:

Sense primer: CACCCATTTCAACCAGTCTA

Antisense primer: AGCTGGCATACGAATCAAT

These nucleotide sequences permit amplification of a fragment of a length of 655 nucleotides, nucleotide 470 to nucleotide 1124, in the nucleotide sequence SEQ ID N° 1.

For each SNP, the PCR product will serve as a template for the minisequencing

The total reaction volume of the PCR reaction is 5 µl per sample.

This reaction volume is composed of the reagents indicated in the following table:

| Supplier | Reference | Reactant | Initial Conc. | Vol. per tube ( µl) | Final Conc. |
|---|---|---|---|---|---|
| Life Technology | Delivered with Taq | Buffer (X) | 10 | 0.5 | 1 |
| Life Technology | Delivered with Taq | $MgSO_4$ (mM) | 50 | 0.2 | 2 |
| AP Biotech | 27-2035-03 | dNTPs (mM) | 10 | 0.1 | 0.2 |
| | On request | Sense Primer (µM) | 10 | 0.1 | 0.2 |
| | On request | Antisense Primer (µM) | 10 | 0.1 | 0.2 |
| Life Technology | 11304-029 | Taq platinum | 5U/µl | 0.02 | 0.1 U/ reaction |
| | | $H_2O$ | Qsp 5 µl | 1.98 | |
| | | DNA (sample) | 2.5 ng/ µl | 2 | 5 ng/ reaction |
| | | Total volume | | 5 µl | |

These reagents are distributed in a black PCR plate having 384 wells provided by ABGene (ref: TF-0384-k). The plate is sealed, centrifuged, then placed in a thermocycler for 384-well plates (Tetrad of MJ Research) and undergoes the following incubation: PCR Cycles: 1 min at 94° C, followed by 36 cycles composed of 3 steps (15 sec. at 94° C, 30 sec. at 56° C, 1 min at 68° C).

2) The PCR amplified product is then purified using two enzymes: Shrimp Alkaline Phosphatase (SAP) and exonuclease I (Exo I). The first of these enzymes permits the dephosphorylation of the dNTPs which have not been incorporated during the PCR amplification, whereas the second eliminates the single stranded DNA residues, in particular the primers which have not been used during the PCR.

This digestion is done by addition, in each well of the PCR plate, of a reactional mixture of 5 µl per sample.

This reactional mixture is composed of the following reagents:

| Supplier | Reference | Reactant | Initial Conc. | Vol. per tube (µl) | Final conc. |
|---|---|---|---|---|---|
| AP Biotech | E70092X | SAP | 1 U/µl | 0.5 | 0.5/ reaction |
| AP Biotech | 070073Z | Exo I | 10 U/µl | 0.1 | 1/ reaction |
| AP Biotech | Supplied with SAP | Buffer SAP (X) | 10 | 0.5 | 1 |
| | | $H_2O$ | Qsp 5 µl | 3.9 | |

(continued)

| Supplier | Reference | Reactant | Initial Conc. | Vol. per tube (µl) | Final conc. |
|---|---|---|---|---|---|
| | | PCR product | | 5 µl | |
| | | Total vol. | | 10 µl | |

Once filled, the plate is sealed, centrifuged, then placed in a thermocycler for 384-well plates (Tetrad of MJ Research) and undergoes the following incubation: Digestion SAP-EXO: 45 min at 37° C, 15 min at 80° C.
The elongation or minisequencing step is then carried out on the product of PCR digested by addition of a reactional mixture of 5 µl per prepared sample.
The minisequencing 3) and the reading steps 4) and interpretation of reading 5) are specific to each SNP t110c, t338a, t363c, c427t, c527a, and g1023a.
All these steps are described hereinafter outlining the specific conditions used for each one of these polymorphisms.

3) Minisequencing

The sequences of the two minisequencing primers necessary for the genotyping were determined in a way to correspond to the sequence of the nucleotides located upstream of the site of a SNP according to the invention. The PCR product that contains the SNP being a double stranded DNA product, the genotyping can therefore be done either on the sense strand or on the antisense strand. The selected primers are manufactured by Life Technologies Inc.
The following table indicates, for each SNP, the sequence of the minisequencing primers that have been tested and the optimal condition retained for the genotyping:

| SNP | Primers tested | Optimal condition retained for the genotyping |
|---|---|---|
| t110c | Sense primer: taatttaatttttaattgtt<br>Antisense primer: tctttttgctttctttatac | antisense primer +<br>ddATP-R110 + ddGTP-Tamra |
| t338a | Sense primer: ctgaaaacccatgtaaagag<br>Antisense primer: tctttttgctttctttatac | sense primer +<br>dTTP-R110 + ddATP-Tamra |
| t363c | Sense primer: aaagaaagcaaaaagagaag<br>Antisense primer: atgcccctgtgttactttct | sense primer +<br>ddTTP-R110 + ddCTP-Tamra |
| c427t | Sense primer: tccctatttaaggctaggca<br>Antisense primer: ttctctgaagaccttgcttt | sense primer +<br>ddTTP-R110 + ddCTP-Tamra |
| c527a | Sense primer: tacaatggccttgacctttg<br>Antisense primer:ccaggagggccaccagtaaa | antisense primer +<br>ddGTP-R110 + ddTTP-Tamra |
| g1023a | Sense primer: gttgtcagagcagaaatcat<br>Antisense primer: gttgacaaagaaaaagatct | sense primer +<br>ddATP-R110 + ddGTP-Tamra |

The minisequencing of the SNPs was first validated over 16 samples, then genotyped over the set of the population of individuals composed of 268 individuals and 10 controls.
The elongation or minisequencing step is then carried out as indicated in the following table:

| Supplier | Reference | Reactant | Initial conc. | Vol. per tube (µl) | Final conc. |
|---|---|---|---|---|---|
| Own preparation | | Elongation Buffer[1](X) | 5 | 1 | 1 |
| Life Technologies | On request | Miniseq Primer (µM) A or B | 10 | 0.5 | 1 |
| AP Biotech | 27-2051 (61,71,81)-01 | ddNTPs[2] (µM) 2 are non labeled | 2.5 of each | 0.25 | 0.125 of each |
| NEN | Nel 472/5 and Nel 492/5 | ddNTPs[2] ( µM) 2 are labeled with Tamra and R110 | 2.5 of each | 0.25 | 0.125 of each |
| AP Biotech | E79000Z | Thermo-sequenase | 3.2 U/µl | 0.125 | 0.4 U/ reaction |
| | | $H_2O$ | Qsp 5 µl | 3.125 | |
| | | digested PCR product | | 10 | |
| | | Total volume | | 15 | |

[1] The 5 X elongation buffer: is composed of 250 mM Tris-HCl pH 9, 250 mM KCl, 25 mM NaCl, 10 mM $MgCl_2$ and 40 % glycerol.
[2] ddNTPs : For the ddNTPs, a mixture of the 4 bases is carried out according to the polymorphism studied. Only the 2 bases of interest (wild-type nucleotide /mutated nucleotide) composing the SNP are labeled, either in Tamra, or in R110. For example:
[3] For SNP c527a, the mixture of ddNTPs is composed of:

- 2.5 µM of ddCTP non-labeled,
- 2.5 µM of ddATP non labeled,
- 2.5 µM of ddTTP (1.875 µM of ddTTP non labeled and 0.625 µM of ddTTP Tamra labeled),
- 2.5 µM of ddGTP (1.875 µM of ddGTP non labeled and 0.625 µM of ddGTP R110 labeled).

For SNP g1023a, the mixture of ddNTPs is composed of:

- 2.5 µM of ddCTP non labeled,
- 2.5 µM of ddTTP non-labeled,
- 2.5 µM of ddGTP (1.875 µM of ddGTP non labeled and 0.625 µM of ddGTP Tamra labeled),
- 2.5 µM of ddATP (1.875 µM of ddATP non labeled and 0.625 µM of ddATP R110 labeled).

Once filled, the plate is sealed, centrifuged, then placed in a thermocycler for 384-well plates (Tetrad of MJ Research) and undergoes the following incubation: Elongation cycles : 1 min. at 93° C, followed by 35 cycles composed of 2 steps (10 sec. at 93° C, 30 sec. at 55° C).

After the last step in the thermocycler, the plate is directly placed on a polarized fluorescence reader of type Analyst® HT of LJL Biosystems Inc. The plate is read using Criterion Host® software by using two methods. The first permits reading the Tamra labeled base by using emission and excitation filters specific for this fluorophore (excitation 550-10 nm, emission 580-10 nm) and the second permits reading the R110 labeled base by using the excitation and emission filters specific for this fluorophore (excitation 490-10 nm, emission 520-10 nm). In the two cases, a dichroic double mirror (R110/Tamra) is used and the other reading parameters are:

Z-height: 1.5 mm
Attenuator: out
Integration time: 100,000 µsec.
Raw data units: counts/sec
Switch polarization: by well
Plate settling time: 0 msec
PMT setup: Smart Read (+), sensitivity 2
Dynamic polarizer: emission
Static polarizer: S

A file result is thus obtained containing the calculated values of mP (milliPolarization) for the Tamra filter and that for the R110 filter. These mP values are calculated starting from intensity values obtained on the parallel plane (*II*) and on the perpendicular plane (⊥) according to the following formula :

$$MP = 1000(II - g_L)/(II + g_L).$$

In this calculation, the value $_L$ is weighted by a factor g. It is a machine parameter that must be determined experimentally beforehand.

4) and 5) Interpretation of the reading and determination of the genotypes.

The mP values are reported on a graph using Microsoft Inc. Excel software, and/or Allele Caller® software developed by LJL Biosystems Inc.

On the abscissa is indicated the mP value of the Tamra labeled base, on the ordinate is indicated the mP value of the R110 labeled base. A strong mP value indicates that the base labeled with this fluorophore is incorporated and, conversely, a weak mP value reveals the absence of incorporation of this base.

Up to three homogenous groups of nucleotide sequences having different genotypes are obtained.

The use of the Allele Caller® software permits, once the identification of the different groups is carried out, direct extraction of the genotype defined for each individual in table form.

Results of the miniseguencing for the SNPs t110c, t338a, t363c, c427t, c527a, and g1023a

After the completion of the genotyping process, the determination of the genotypes of the individuals of the population of individuals for the SNPs studied here was carried out using the graphs described above.

For SNP t110c, the genotype is in theory either homozygote TT, or heterozygote TC, or homozygote CC in the tested individuals. In reality, and as shown below, the homozygote genotype CC is not detected in the population of individuals.

For SNP t338a, the genotype is in theory either homozygote TT, or heterozygote TA, or homozygote AA in the tested individuals. In reality, and as shown below, the homozygote genotype AA is not detected in the population of individuals.

For SNP t363c, the genotype is in theory either homozygote TT, or heterozygote TC, or homozygote CC in the tested individuals. In reality, and as shown below, the homozygote genotype CC is not detected in the population of individuals.

For SNP c427t, the genotype is in theory either homozygote CC, or heterozygote CT, or homozygote TT in the tested individuals. In reality, and as shown below, the homozygote genotype TT is not detected in the population of individuals.

For SNP c527a, the genotype is in theory either homozygote CC, or heterozygote CA, or homozygote AA in the tested individuals. In reality, and as shown below, the homozygote genotype AA is not detected in the population of individuals.

For SNP g1023a, the genotype is in theory either homozygote GG, or heterozygote GA, or homozygote AA in the tested individuals. In reality, and as shown below, the homozygote genotype AA is not detected in the population of individuals.

The results of the distribution of the determined genotypes in the population of individuals and the calculation of the different allelic frequencies for the 6 SNPs studied are presented in the following tables:

| | | t110c | | | | | | | | | |
|---|---|---|---|---|---|---|---|---|---|---|---|
| **Phylogenic Population** | **Total** | ***f*** | ***(95% CI)*** | ***TT*** | ***%*** | ***TC*** | ***%*** | ***CC*** | ***%*** | ***Total*** |
| African American | 50 | **8.0** | **(2.7, 13.3)** | 42 | 84.0 | 8 | 16.0 | | | 50 |
| Amerind | 15 | | | 15 | 100 | | | | | 15 |
| Caribbean | 10 | | | 10 | 100 | | | | | 10 |
| European Caucasoid | 99 | **1.0** | **(0, 2.4)** | 97 | 98.0 | 2 | 2.0 | | | 99 |
| Mexican | 10 | | | 10 | 100 | | | | | 10 |
| Non-European Caucasoid | 37 | | | 37 | 100 | | | | | 37 |
| Northeast Asian | 20 | | | 20 | 100 | | | | | 20 |
| South American | 10 | **5.0** | **(0, 14.6)** | 9 | 90.0 | 1 | 10.0 | | | 10 |
| Southeast Asian | 17 | | | 17 | 100 | | | | | 17 |
| ***Total*** | ***268*** | ***2.1*** | ***(0.9, 3.3)*** | ***257*** | ***95.9*** | ***11*** | ***4.1*** | | | ***268*** |

| Phylogenic Population | Total | t338a | | | | | | | | | |
|---|---|---|---|---|---|---|---|---|---|---|---|
| | | f | (95% CI) | TT | % | TA | % | AA | % | Total |
| African American | 50 | 17.0 | (9.4, 24.6) | 31 | 66.0 | 16 | 34.0 | | | 47 |
| Amerind | 15 | | | 15 | 100 | | | | | 15 |
| Caribbean | 10 | 5.0 | (0, 14.6) | 9 | 90.0 | 1 | 10.0 | | | 10 |
| European Caucasoid | 99 | 0.5 | (0, 1.5) | 98 | 99.0 | 1 | 1.0 | | | 99 |
| Mexican | 10 | | | 10 | 100 | | | | | 10 |
| Non-European Caucasoid | 37 | 1.4 | (0, 4.0) | 36 | 97.3 | 1 | 2.7 | | | 37 |
| Northeast Asian | 20 | | | 20 | 100 | | | | | 20 |
| South American | 10 | | | 10 | 100 | | | | | 10 |
| Southeast Asian | 17 | | | 17 | 100 | | | | | 17 |
| *Total* | 268 | 3.6 | (2.0, 5.2) | 246 | 92.8 | 19 | 7.2 | | | 265 |

| Phylogenic Population | Total | t363c | | | | | | | | |
|---|---|---|---|---|---|---|---|---|---|---|
| | | f | (95% CI) | TT | % | TC | % | CC | % | Total |
| African American | 50 | | | 48 | 100 | | | | | 48 |
| Amerind | 15 | | | 15 | 100 | | | | | 15 |
| Caribbean | 10 | | | 10 | 100 | | | | | 10 |
| European Caucasoid | 99 | 0.5 | (0, 1.5) | 97 | 99.0 | 1 | 1.0 | | | 98 |
| Mexican | 10 | | | 10 | 100 | | | | | 10 |
| Non-European Caucasoid | 37 | | | 36 | 100 | | | | | 36 |
| Northeast Asian | 20 | | | 20 | 100 | | | | | 20 |
| South American | 10 | | | 10 | 100 | | | | | 10 |
| Southeast Asian | 17 | | | 17 | 100 | | | | | 17 |
| *Total* | 268 | 0.2 | (0, 0.6) | 263 | 99.6 | 1 | 0.4 | | | 264 |

| Phylogenic Population | Total | c427t | | | | | | | | |
|---|---|---|---|---|---|---|---|---|---|---|
| | | f | (95% CI) | CC | % | CT | % | TT | % | Total |
| African American | 50 | | | 50 | 100 | | | | | 50 |
| Amerind | 15 | | | 15 | 100 | | | | | 15 |
| Caribbean | 10 | | | 10 | 100 | | | | | 10 |
| European Caucasoid | 99 | | | 99 | 100 | | | | | 99 |
| Mexican | 10 | | | 10 | 100 | | | | | 10 |
| Non-European Caucasoid | 37 | | | 37 | 100 | | | | | 37 |
| Northeast Asian | 20 | | | 20 | 100 | | | | | 20 |
| South American | 10 | 5.0 | (0, 14.6) | 9 | 90.0 | 1 | 10.0 | | | 10 |
| Southeast Asian | 17 | | | 17 | 100 | | | | | 17 |
| *Total* | 268 | 0.2 | (0, 0.6) | 267 | 99.6 | 1 | 0.4 | | | 268 |

| Phylogenic Population | Total | c527a (A6D) | | | | | | | | |
|---|---|---|---|---|---|---|---|---|---|---|
| | | *f* | *(95% CI)* | *CC* | *%* | *CA* | *%* | *AA* | *%* | *Total* |
| African American | *50* | | | *50* | *100* | | | | | *50* |
| Amerind | *15* | | | *15* | *100* | | | | | *15* |
| Caribbean | *10* | ***5.0*** | ***(0, 14.6)*** | *9* | *90.0* | *1* | *10.0* | | | *10* |
| European Caucasoid | *99* | | | *95* | *100* | | | | | *95* |
| Mexican | *10* | | | *10* | *100* | | | | | *10* |
| Non-European Caucasoid | *37* | | | *37* | *100* | | | | | *37* |
| Northeast Asian | *20* | | | *20* | *100* | | | | | *20* |
| South American | *10* | | | *10* | *100* | | | | | *10* |
| Southeast Asian | *17* | | | *17* | *100* | | | | | *17* |
| ***Total*** | ***268*** | ***0.2*** | ***(0, 0.6)*** | ***263*** | ***99.6*** | ***1*** | ***0.4*** | | | ***264*** |

| Phylogenic Population | Total | g1023a (M171I) | | | | | | | | |
|---|---|---|---|---|---|---|---|---|---|---|
| | | *f* | *(95% CI)* | *GG* | *%* | *GA* | *%* | *AA* | *%* | *Total* |
| African American | *50* | | | *50* | *100* | | | | | *50* |
| Amerind | *15* | | | *14* | *100* | | | | | *14* |
| Caribbean | *10* | ***5.0*** | ***(0, 14.6)*** | *9* | *90.0* | *1* | *10.0* | | | *10* |
| European Caucasoid | *99* | | | *97* | *100* | | | | | *97* |
| Mexican | *10* | | | *10* | *100* | | | | | *10* |
| Non-European Caucasoid | *37* | | | *37* | *100* | | | | | *37* |
| Northeast Asian | *20* | | | *20* | *100* | | | | | *20* |
| South American | *10* | | | *10* | *100* | | | | | *10* |
| Southeast Asian | *17* | | | *17* | *100* | | | | | *17* |
| ***Total*** | ***268*** | ***0.2*** | ***(0, 0.6)*** | ***264*** | ***99.6*** | ***1*** | ***0.4*** | | | ***265*** |

In the above tables,

- N represents the number of individuals,
- % represents the percentage of individuals in the specific sub-population,
- the allelic frequency represents the percentage of the mutated allele in the specific sub-population,
- 95 % IC represents the minimal and maximal interval of confidence at 95 %.

It is necessary to specify that, for example for the c527a SNP that has been genotyped in antisense, the allele g read in antisense corresponds to the allele c read in sense, and to the presence of a alanine at position 6 of the amino acid sequence of the immature IFNα-2 protein and therefore that the allele t read in antisense corresponds to the allele a read in sense corresponding to an aspartic acid for this position in the sequence of the corresponding protein.

By examining these results by phylogenic population, and by SNP, it is observed that:

- for SNP t110c, the 11 heterozygote individuals TC come from the phylogenic populations African-American, European Caucasoid, and South American in the population of individuals.
- for SNP t338a, 19 heterozygote individuals TA are found, they come from the phylogenic populations African American, Caribbean, European Caucasoid, Non-European Caucasoid in the population of individuals.
- for SNP t363c, the only heterozygote individual TC comes from the phylogenic population European Caucasoid in the population of individuals.
- for SNP c427t, the only heterozygote individual CT comes from the phylogenic population South American in the population of individuals.
- for SNP c527a, the only heterozygote individual CA comes from the phylogenic population Caribbean in the population of individuals.
- for SNP g1023a, the only heterozygote individual GA comes from the phylogenic population Caribbean in the population of individuals.

Example 5. Expression of natural wild-type IFNα-2 and M148I mutated IFNα-2 in bacteria

a) Cloning of the natural wild-type IFNα-2 and mutated (M148I) IFNα-2 in the prokaryotic expression vector pTrc/His-topo

The nucleotide sequences coding for the natural wild-type and mutated IFNα-2 protein are amplified by PCR.
The PCR primers permitting such an amplification are:

Sense primer: CACCCATTTCAACCAGTCTA

Antisense primer: AGCTGGCATACGAATCAAT

The PCR products are inserted in the prokaryotic expression vector pTrc/His-Topo under the control of the Trc hybrid promoter, inducible by IPTG (Iso-Propyl-Thio-Galactoside), by TOPO™-*cloning* (Invitrogen Corp.).
This vector permits the heterologous expression of eukaryotic proteins in the bacteria thanks to the presence of a mini-cistronic unit.
The wild-type protein and the mutated protein are produced in the form of fusion proteins carrying an N-terminal extension composed of a 6-histidine tail and the epitope for a specific antibody.
It is possible to cleave this additional region by using the Enterokinase endoprotease.
After checking of the nucleotide sequence of the region of the vector coding for the recombinant proteins, the Top 10 strain of *E. coli* (Invitrogen) is transformed with these recombinant expression vectors.

b) Heterologous expression in *E. coli* and purification of the natural wild-type IFNα-2 and mutated M148I mutated polyhistidine IFNα-2

Two saturated pre-cultures of 100 ml of LBA medium (Luria Bertoni + 100 μg/mL ampicillin) containing, respectively, a clone coding for wild-type IFNα-2 or that coding for M148I IFNα-2, were carried out for 24 hours at 30°C at an agitation of 200 rotations per minute (rpm). After 24 hours of growth, the cultures were used to inoculate, at 1/10, 900 mL of LBA medium (preinculbated over-night at 37°C).
When the second set of cultures reaches a cellular density corresponding to an optical density of 0.8 measured at a wavelength of 600 nm, the expression of the poly-hystidine proteins is then induced by the addition of IPTG at a final concentration of 1 mM and the culture is kept for 5 hours at 30 °C with an agitation at 200 rpm.
The pellet of bacteria obtained after centrifugation at 4000 x g, 30 min, 4 °C, is resuspended in 25 mL of buffer A (Tris 50 mM, pH 8, NaCl 50 mM, imidazole 10 mM, PMSF 0.1 mM pH 8).
A preincubation of 30 min in ice in the presence of 0.5 mg/mL of lysozyme and 20 units of DNase I precedes a sonication carried out in three steps with control of the temperature of the sample (a step delivered 240 Watt per impulse of 10 sec with 10 sec stop, for 1 min). The cell suspension is then clarified by centrifugation at 15,000 x g for 30 min at 4 °C.
The centrifugation supernatant is next filtered on 0.22 micrometer-filter.
The polyhistidine proteins present are then purified by HPLC on HiTrap™ Nickel Affinity resin (Amersham Pharmacia Biotech) previously equilibrated in 50 mM Tris, 300 mM NaCl pH 8.0 (Buffer B). After copiously washing the column with 1M NaCl in 50 mM Tris pH 8.0, the elution of the proteins was induced by a linear gradient of imidazole with concentrations ranging from 0.01 to 0.25 M in buffer B.
The presence of the polyhistidine protein in the collected fractions is verified, on the one hand by SDS PAGE electrophoresis and on the other hand by immunodetection with the aid of a specific antibody directed against the N-terminal end of the fusion protein.
At this stage, the protein of interest is pure up to 80%.
The last step of the purification consists of a separation of the proteins on an ion-exchange chromatography column.
The fractions containing the fusion protein are injected on an anion-exchange column (MiniQ PE 4.6/50, Pharmacia) that was previously equilibrated in buffer 50 mM Tris pH 8. The elution of the proteins is carried out by the passage of an NaCl gradient ranging from 0 to 500 mM, in buffer 50 mM Tris pH 8.
The purity of the protein of interest is estimated on the SDS/PAGE gel and the protein concentrations were measured by BCA assay (bicinchoninic acid and copper sulfate, Sigma).
The purified wild-type and mutated IFNα-2 proteins containing the N-terminal polyhistidine end are used in the test on Daudi cells consisting of measurement of the antiproliferative activity of these two forms of IFNα-2 on the cell growth of the Daudi cell line.

Example 6. Expression of natural wild-type IFNα-2 and M148I mutated IFNα-2 in yeast

a) Cloning of the natural wild-type IFNα-2 and M148I mutated IFNα-2 in the eukaryote expression vector pPicZα-topo

The nucleotide sequences coding for the mature part of the natural wild-type IFNα-2 and M148I mutated IFNα-2 are amplified by PCR.

The PCR primers permitting such an amplification are:

Sense primer: TGTGATCTGCCTCAAACCCACAG

Antisense primer: TCATTCCTTACTTCTTAAACTTTCTTGC

The PCR products are inserted in the eukaryote expression vector pPicZα-TOPO under the control of the hybrid promoter AOX1 inducible by methanol (TOPO™-*cloning*; Invitrogen Corp.).

This vector permits the heterologous expression of eukaryote proteins in the yeast *Pichia pastoris.*

After checking of the nucleotide sequence of the region of the vector coding for the recombinant proteins, the vector is linearized by the Pme1 restriction enzyme, and the *P. pastoris* yeast strain (Invitrogen) is transformed with these recombinant expression vectors.

b) Heterologous expression in *P. pastoris* and purification of the natural wild-type IFNα-2 and M148I mutated IFNα-2 proteins

Two saturated pre-cultures of 50 ml of BMGY medium (2% Peptone, 1% yeast extract, 1.34% YNB, 1% Glycerol, 100 mM potassium phosphate, 0.4 mg/Liter biotin pH 6.0) containing a clone coding for natural wild-type IFNα-2 or that coding for M1481 mutated IFNα-2, were carried out for 24-48 hours at 30°C at an agitation of 200 rotations per minute (rpm).

When the culture reaches a saturating cellular density (corresponding to an optical density of 12 measured at a wavelength of 600 nm), it is used to inoculate, at 5 OD/ml, 250 ml of BMMY medium (2% Peptone, 1% yeast extract, 1.34% YNB, 0.5% Methanol, 100 mM potassium phosphate, 0.4 mg/Liter biotin pH 6.0).

The expression of the protein is then induced by methanol at a final concentration of 1%, for 24 hours at 30 °C, with an agitation of the culture flask at 180 rpm.

Due to the presence of the signal peptide sequence of the "alpha factor", upstream of the coding sequence, the proteins are secreted by the yeasts in the culture medium. The alpha factor is naturally cleaved during the processing.

The suspension is centrifuged and the protein is purified by HPLC starting from the obtained supernatant.

In a pre-started step, an ultrafiltration (Labscale, cut-off 5000Da, Millipore) followed by a dialysis permits a ten times concentration of the yeast supernatant in a buffer of 50 mM Tris-Cl pH 9.0, NaCl 25 mM.

The first chromatographic step permits protein recovery by affinity on a blue sepharose column (Amersham Pharmacia). The presence of the protein in the collected fractions is verified, on the one hand by electrophoresis of SDS PAGE type and on the other hand by immuno-detection by a specific antibody directed against the IFNα-2 protein. At this step, the purity of the protein of interest is higher than 75%.

In a second purification step, a gel filtration permits buffer exchange of the collected fractions corresponding to IFNα-2 proteins against 50 mM Tris pH 9.0, NaCl 25 mM.

The last step of the purification consists of a separation of the proteins on an ion exchange chromatography column.

The fractions containing the recombinant protein are injected on an anion exchange column (ResourceQ 6.0 mL, Pharmacia) equilibrated beforehand in Tris 50 mM pH 9, NaCl 25 mM buffer. The elution of the proteins is carried out by the migration of a gradient between 0,025 and 1 M NaCl in the Tris 50 mM pH 9 buffer.

The purity of the protein of interest is estimated on SDS/PAGE gel and the protein concentrations were measured by densitometry (Quantity one, Biorad) and BCA assay (bicinchoninic acid and copper sulfate, Sigma).

Purified natural wild-type IFNα-2 and M148I mutated IFNα-2 proteins obtained according to the protocols described in examples 5 and 6, eventually scaled-up to produce higher amount of proteins, are used for the functional tests described below.

Example 7. Evaluation of the capacity of natural wild-type and M148I mutated IFNα-2 to activate signal transduction in the breast carcinoma cell line MCF-7

The interferons are known to act through signaling pathways involving the JAK (Janus Kinase) and the STAT (Signal Transducers and Activators of Transcription) proteins. The binding of interferon to its receptor induces phosphorylation of the JAK proteins which in turn activate by phosphorylation the STAT proteins. Activated STAT proteins translocate to the nucleus where they bind to interferon response elements on gene promoters, which stimulates transcription of the respective genes. To study the signaling pathways initiated by interferon, the reporter gene technique was used. The procedure is described below.

The breast carcinoma cells MCF-7 (ECACC) were seeded at a density of $1.10^4$ cells/well in 96-well plates in RPMI supplemented with 10% fetal calf serum for 24 hours. Cells were then transfected for 6 hours with a reporter gene construct (pISRE-Luc) coding for the Firefly Luciferase placed under the control of the Interferon-Stimulated Response Element (Clontech) using Superfect (Qiagen) according to the manufacturer's instructions. Then, culture media were changed and cells were incubated over-night in a $CO_2$ incubator at 37°C after which they were stimulated with various doses of wild-type or mutated IFNα-2 protein for 6 hours at 37°C. After stimulation, culture media were discarded and replaced with 100µl/well of Phosphate Buffered Saline (PBS)/1mM $MgCl_2$. Luciferase activity was measured in a MicroBeta counter (Perkin-Elmer) following addition of 100µl/well of the substrate Luclite-Plus (Packard).

Results are expressed as the percentage of maximal stimulation of the Luciferase activity. The measurements were performed three times, and the values given here represent the means of the triplicate determinations.

The ability of the wild-type IFNα-2 or M148I mutated IFNα-2 to trigger the signal transduction cascades is based on the measurements of their Efficacy Doses at 50% (EC50's) corresponding to their respective concentrations stimulating 50% of the Luciferase activity (the maximal stimulation is considered as being 100% activity).

The average EC50 value measured for the wild-type IFNα-2 is 12.33 pM.

The average EC50 value measured for the M148I mutated IFNα-2 is 122 pM.

Thus, the ratio corresponding to the EC50 value for the mutated protein over the EC50 value for the wild-type protein reaches 9.91 (with a standard deviation of 3.4).

Consequently, this test demonstrates that the biological activity of M148I mutated IFNα-2 is 10 times less than that of wild-type IFNα-2 based on its capacity to activate the interferon signaling pathway.

Example 8. Evaluation of immunomodulatory activity of natural wild-type IFNα-2 and M148I mutated IFNα-2

IFNs type I (IFN alpha and IFN beta) are able to modulate certain functions of the immune system. They have been demonstrated to increase the dendritic cells (DC) maturation: increase in the expression of MHC class I (HLA ABC) and II (HLA ADR) molecules, increase in the expression of the molecules involved in the co-stimulation of the T-lymphocytes, CD80, CD86 and CD83 molecules and increase in the stimulating function of T-lymphocyte.

a) Effect of natural wild-type IFNα-2 and M148I mutated IFNα-2 on dendritic cell maturation

Immunomodulatory activity of wild-type IFNα-2 and M148I mutated IFNα-2 was first investigated on dendritic cells maturation.

To do so, dendritic cells were first generated from adult peripheral blood monocytes cultivated in the presence of GM-CSF and IL-4 cytokines. After purification using a CD14+ cells purification kit, these dendritic cells were placed in presence of 100 ng/mL of wild-type IFNα-2 or M148I mutated IFNα-2 and their phenotype was determined by FACS analysis aiming at looking for the expression of the MHC class I and II molecules and the CD40, CD80, CD86, CD83, and CD1a markers. The maturation state of these dendritic cells has also been compared to that obtained without IFNα-2 treatment, to provide a control with non-stimulated dendritic cells.

The median value of the measures of fluorescence intensity for each marker and for the three experimental conditions, expressed as arbitrary unit, are presented in the following table :

| | **HLA** ABC | **HLA** ADR | **CD40** | **CD80** | **CD86** | **CD83** | **CD1a** |
|---|---|---|---|---|---|---|---|
| No stimulation | 64 | 133 | 24 | 25 | 14 | 15 | 26 |
| Wild-type IFNα-2 | 87 | 281 | 331 | 76 | 45 | 15 | 155 |
| M148I IFNα-2 | 86 | 55 | 149 | 40 | 15 | 16 | 202 |

b) Effect of natural wild-type IFNα-2 and M148I mutated IFNα-2 on cytokine release by T lymphocytes

Immunomodulatory activity of wild-type IFNα-2 and M148I mutated IFNα-2 was also investigated by measuring cytokine release by T lymphocytes placed in presence of wild-type IFNα-2 and M148I mutated IFNα-2 and with or without a strong antigen (SEB) in order to mimic an immune response against an aggression.

To do so, peripheral blood mononuclear cells (PBMC) were isolated from healthy donors and stimulated for 16 hours in an appropriate medium containing anti-CD3 and anti-CD28 antibodies or SEB. In each culture was added 4 µg/mL of wild-type IFNα-2 or M148I mutated IFNα-2. After stimulation, T lymphocytes were extracellularly labelled with anti-CD3, anti-CD4 and anti-CD69 antibodies or anti-CD3, anti-CD8 and anti-CD69 antibodies, and intracellularly labelled with specific antibodies directed against Th1-type cytokines (IFN-gamma) or Th2-type cytokines (IL-10).

Fluorescent cells were analysed using FACScalibur and CellQuest software.

The results obtained indicate that both M148I mutated IFNα-2 and wild-type IFNα-2 do not stimulate IL-10 and IFN-gamma release and, thus, do not activate T lymphocytes in absence of SEB. In contrast, both M148I mutated IFNα-2 and wild-type IFNα-2 stimulate cytokines (IL-10 and IFN-gamma) release by SEB-activated T-lymphocytes as shown in the table below. This table represents the cytokine release by T-lymphocytes in presence of SEB, expressed as percentage of the CD4+ CD69+ cells or CD8+ CD69+ cells for the CD4+ T-lymphocytes and CD8+ T-lymphocytes, respectively, and the percentage of CD69+ cells on total cells.

| T-lymphocyte | | **IFN gamma** | **IL-10** | **CD69+ cells/total** |
|---|---|---|---|---|
| CD4+ CD69+ | Negative control | 11.9 | 7.5 | 1.26 |
| | Wild-type IFNα-2 | 19.6 | 24.68 | 2.7 |
| | M148I mutated IFNα-2 | 24.03 | 10.3 | 2.6 |
| CD8+ CD69+ | Negative control | 8.73 | 0.65 | 4.69 |
| | Wild-type IFNα-2 | 16.37 | 4.26 | 10.02 |
| | M148I mutated IFNα-2 | 24.84 | 3.4 | 10.51 |

c) Effect of natural wild-type IFNα-2 and M148I mutated IFNα-2 on cytokine release by monocytes

Finally, immunomodulatory activity of wild-type IFNα-2 and M148I mutated IFNα-2 was investigated by measuring cytokine release by monocytes in absence or in presence of a bacterial toxic agent (LPS).

To do so, human peripheral blood mononuclear cells (PBMC) were isolated from healthy donors and their phenotype was analyzed to determine the relative amount of CD64+ CD4dim cells (CD64 and CD4dim are markers for blood monocytes). After an over-night culture, these PBMC were incubated in the culture medium alone (not stimulated cells) or in presence of LPS (stimulated cells). In each culture, 4 µg/ml of wild-type IFNα-2 or M148I mutated IFNα-2 was added. After culture, cells were extracellularly labelled with anti-CD64 and anti-CD4dim, and intracellularly labelled with specific antibodies directed against Th1-type cytokines (TNF-alpha), IL-12 and IL-10.

Fluorescent cells were analyzed using FACScalibur and CellQuest software.

The results obtained indicate that both M148I mutated IFNα-2 and wild-type IFNα-2 do not stimulate cytokines (IL-10, IL-12 and TNF-alpha) release in absence of LPS. In contrast, in presence of LPS, both M148I mutated IFNα-2 and wild-type IFNα-2 stimulate cytokines (IL-10, IL-12 and TNF-alpha) release by monocytes as shown in the table below. This table represents cytokine release by monocytes in presence of LPS, expressed as percentage of the CD64+ CD4dim cells, and the percentage of CD4dim CD64+ cells on total cells.

| | **IL-10** | **IL-12** | **TNF-α** | **CD4dim CD64+ cells/total** |
|---|---|---|---|---|
| No stimulation | 16.21 | 8.52 | 13.88 | 3.1 |
| Wild-type IFNα-2 | 49.34 | 34.48 | 50.87 | 2.71 |
| M148I mutated IFNα-2 | 42.4 | 27.86 | 60.14 | 2.85 |

Example 9. <u>Evaluation of *in vitro* antiproliferative activity of natural wild-type IFNα-2 and M148I mutated IFNα-2</u>

a) on the human lymphoblasts of Daudi Burkitt's cell line proliferation

These tests are carried out on two different types of IFNα-2, namely: natural wild-type IFNα-2 and M148I

IFNα-2. Cells (human Daudi Burkitt's lymphoma cell line, hereinafter called "Daudi cells") cultivated beforehand in a RPMI 1640 medium (supplemented with 10% fetal calf serum and 2 mM of L-Glutamine) are inoculated in 96-well plates at the cellular density of $4.10^4$ cells/well.

In each well, Daudi cells are placed in contact of increasing concentrations of either natural wild-type or mutated IFNα-2.

For each of the wild-type and mutated IFNα-2, final concentrations of 0.003 pM to 600 nM are tested.

Eight different cultures, and therefore different measurements, are carried out in parallel for both proteins and for each concentration.

The Daudi cells are then incubated for 66 h at 37 °C under 5% $CO_2$ after which the Uptiblue reagent (Uptima) is added to the cultures. The rate of cell proliferation is quantified by measuring the fluorescence emitted at 590nm (excitation 560nm) after an additional period of incubation of 4 hours.

The antiproliferative activity of the wild-type IFNα-2 or M148I mutated IFNα-2 is based on the measurements of the IC50 corresponding to the concentration of IFNα-2 inhibiting 50% of the cell growth.

The results obtained are illustrated in Figure 3.

For each concentration of wild-type and mutated (M148I) proteins, the points represented on the graph are the mean of four measurements performed on the four cultures made in parallel for each of the proteins and concentrations.

The average IC50 value measured for the wild-type IFNα-2 is 0.3, whereas the average IC50 value measured for the mutated M148I IFNα-2 is 5.0. Thus, the ratio corresponding to the value of the IC50 of the mutated protein over the value of the natural wild-type protein reaches 15.3.

This test demonstrates that the cellular antiproliferative activity is greatly decreased in the case of M148I mutated IFNα-2 by comparison with wild-type IFNα-2.

b) on the TF-1 erythroleukemia cell line

The effect of the wild-type and mutant IFNα-2 was also evaluated on TF-1 erythroleukemia cell line. To do so, TF-1 cells were placed in contact of increasing concentrations of either natural wild-type or mutated IFNα-2 (0.001 to 1000 ng/mL) and the cell proliferation measured.

The results are expressed as the IC30 corresponding to the IFNα-2 concentration inhibiting proliferation of 30% of cells. The IC30 measured for the wild-type IFNα-2 is 0.66, and the IC30 measured for the mutant IFNα-2 is 3.09. These data indicate that the M148I mutated IFNα-2 has a weak antiproliferative effect on TF-1 cells, and this effect is similar to that of wild-type IFNα-2, suggesting that the M148I mutated IFNα-2 hematologic toxicity is not superior than that of wild-type IFNα-2.

<u>Example 10. Evaluation of the antiviral activity of natural wild-type IFNα-2 and M148I mutated IFNα-2</u>

The IFNs play an important role in the antiviral defence. The IFN antiviral activity is partly due to IFNs induced enzymatic systems, such as:

- The 2'5' oligoadenylate synthetase, an enzyme which catalyzes the adenosine oligomere synthesis. These oligomeres activate the RNase L, an endoribonuclease which destroy the viral RNA once activated.
- The Mx proteins (GTPases) which inhibit the synthesis and/or the maturation of viral transcripts. This activity is mainly exerted on the influenza virus.
- The PKR protein (or p68 kinase) which is activated by the doublestranded RNA. The activated PKR inhibits proteic synthesis.

The IFNs antiviral activity is also induced by other mechanisms such as, in the case of retroviruses, the inhibition of viral particles entry into the cells, the replication, the binding, the exit of the particles and the infective power of viral particles.

Finally, the IFNs exert an indirect antiviral activity by modulating certain functions of the immune system, in particular by favouring the response to cellular mediation (increase of the MHC class I and II molecules, increase of IL-12 and IFN-gamma production, increase of the CTL activities...).

The antiviral activity of M148I mutated IFNα-2 has been evaluated both *in vitro* in cell culture and *in vivo* in mouse model.

a) Antiviral activity *in vitro* in cell culture

This assay permits evaluation of the antiviral activity of natural wild-type IFNα-2 and M148I mutated IFNα-2

in cell culture using the vesicular stomatitis virus (VSV).

To do so, WISH human epithelial cells are cultivated for 24 hours in the presence of decreasing concentrations of M148I mutated IFNα-2 and wild-type IFNα-2. Then, the cells are infected by the virus of vesicular stomatitis (VSV) during 24 to 48 additional hours and the cell lysis is measured.

The antiviral effect of the different IFNα tested is determined by comparing the IC50 value corresponding to the IFN concentration inhibiting 50% of cell lysis induced by the VSV.

A similar experiment has been carried out two times, and the average results indicate that the IC50 reaches 4 ng/ml in the case of wild-type IFNα-2 and 22 ng/ml in the case of M148I mutated IFNα-2. Thus, in cell culture infected with VSV, the M148I mutated IFNα-2 has a lower antiviral activity than the wild-type IFNα-2.

b) Antiviral activity *in vivo* in mouse model

This test *in vivo* is performed in EMCV (Encephalomyocarditis virus) mouse model.

Human IFNs nevertheless exhibit dose-dependent antiviral activity in the mouse which is in general 100 to 1,000 fold less than that exhibited by the same amount of mouse IFN (Meister et al. (1986). J. Gen. Virol. 67, 1633-1644).

Intraperitoneal injection of mice with Encephalomyocarditis virus (EMCV) gives rise to a rapidly progressive fatal disease characterized by central nervous system involvement and encephalitis (Finter NB (1973). Front Biol. 2: 295-360). Mouse and human interferon-alpha have both been shown to be effective in protecting mice against lethal EMCV infection (Tovey and Maury (1999). J. IFN Cytokine Res. 19: 145-155).

Groups of 20, six-week old Swiss mice were infected intraperitoneally (ip) with 100 x $LD_{50}$ EMCV and treated one hour later, and then once daily for 3 days thereafter with 2 µg of M148I mutated IFNα-2 and wild-type IFNα-2 preparations. A control group was performed with animals having been treated with excipient only. The animals were followed daily for survival for 21 days.

Results are presented in Figure 4 and indicate that the relative survival rate of the mice which have been treated with M148I mutated IFNα-2 is much higher than the survival rate of the non-treated mice but remains similar to that observed for the mice which have been treated with wild-type IFNα-2.

Thus, this test demonstrates that the antiviral activity in EMCV mouse model of M148I mutated IFNα-2 is similar to that of the wild-type IFNα-2.

All of these results demonstrate that despite some similar features with wild-type IFNα-2, M148I mutated IFNα-2 possesses unique biological properties.

## Claims

**1.** Isolated polynucleotide comprising:

a) a nucleotide sequence having at least 80 % identity with the sequence SEQ ID N° 1 or its coding sequence, it being understood that this nucleotide sequence contains at least one of the following coding SNPs: c527a or g1023a, or
b) a nucleotide sequence complementary to a nucleotide sequence under a).

**2.** Isolated polynucleotide comprising:

a) the nucleotide sequence SEQ ID N° 1 or its coding sequence, it being understood that each one of these sequences comprises at least one of the following coding SNPs: c527a, g1023a, or
b) a nucleotide sequence complementary to a nucleotide sequence under a).

**3.** Polynucleotide according to any one of claims 1 to 2, **characterized in that** it consists of the sequence SEQ ID N° 1 or its coding sequence, it being understood that each one of the sequences contains at least one of the following coding SNPs: c527a, g1023a.

**4.** Polynucleotide according to any one of claims 1 to 3, **characterized in that** the nucleotide sequence a) contains a single coding SNP selected from the group consisting of: c527a and g1023a.

**5.** Isolated polynucleotide comprising:

a) the nucleotide sequence SEQ ID N° 1 or if necessary its coding sequence, it being understood that each one of these sequences contains at least one of the following SNPs: 96-100del(aattt), t110c, 139-144del(acttta), t338a, t363c, c427t, c1047t, or
b) a nucleotide sequence complementary to a nucleotide sequence under a).

**6.** Isolated polynucleotide consisting of a part of:

a) a nucleotide sequence SEQ ID N° 1 or if necessary its coding sequence, it being understood that each one of these sequences contains at least one of the following SNPs: 96-100del(aattt), t110c, 139-144del(acttta), t338a, t363c, c427t, c527a, g1023a, c1047t, or
b) a nucleotide sequence complementary to a nucleotide sequence under a);

said isolated polynucleotide being composed of at least 10 nucleotides.

**7.** Isolated polynucleotide, **characterized in that** it codes for a polypeptide comprising:

a) the amino acid sequence SEQ ID N° 2, or
b) the amino acid sequence comprising the amino acids included between positions 24 and 188 of the amino acid sequence SEQ ID N° 2;

it being understood that each one of the amino acid sequences under a) and b) contains at least one of the following coding SNPs: A6D, M171I.

**8.** Polynucleotide according to claim 7, **characterized in that** it codes for a polypeptide containing a single coding SNP selected from the group consisting of: A6D and M171I.

**9.** Use of all or part of:

a) a polynucleotide having 80 to 100 % identity with the nucleotide sequence SEQ ID N°1, and/or
b) a polynucleotide according to any one of claims 1 to 8;

in order to identify, hybridize and/or amplify all or part of a polynucleotide having 80 to 100 % identity with the nucleotide sequence SEQ ID N° 1 or if necessary its coding sequence, it being understood that each one of these sequences contains at least one of the following SNPs: 96-100del(aattt), t110c, 139-144del(acttta), t338a, t363c, c427t, c527a, g1023a, c1047t.

**10.** Use of all or part of:

a) a polynucleotide having 80 to 100 % identity with the nucleotide sequence SEQ ID N°1, and/or
b) a polynucleotide according to any one of claims 1 to 8;

for the genotyping of all or part of a polynucleotide having 80 to 100 % identity with the nucleotide sequence SEQ ID N° 1 or if necessary its coding sequence, it being understood that each one of these sequences contains at least one of the following SNPs: 96-100del(aattt), t110c, 139-144del(acttta), t338a, t363c, c427t, c527a, g1023a, c1047t.

**11.** Use according to claim 10, in which the genotyping is carried out by minisequencing.

**12.** Recombinant vector comprising a polynucleotide according to one of claims 1 to 8.

**13.** Host cell comprising a recombinant vector according to claim 12.

**14.** Process for the preparation of a polypeptide, **characterized in that** a host cell according to claim 13 is cultivated in a culture medium and said polypeptide is isolated from the culture medium.

**15.** Isolated polypeptide comprising an amino acid sequence having at least 80 % identity with:

a) the amino acid sequence SEQ ID N° 2, or
b) the amino acid sequence containing the amino acids included between positions 24 and 188 of the amino acid sequence SEQ ID N° 2;

it being understood that each one of the amino acid sequences under a) and b) contains at least one of the following coding SNPs: A6D, M171I.

**16.** Polypeptide according to claim 15, **characterized in that** it comprises:

a) the amino acid sequence SEQ ID N° 2, or
b) the amino acid sequence containing the amino acids included between positions 24 and 188 of the amino acid sequence SEQ ID N° 2;

it being understood that each one of the amino acid sequences under a) and b) contains at least one of the following coding SNPs: A6D, M171I.

**17.** Polypeptide according to any one of claims 15 and 16, **characterized in that** it consists of:

a) the amino acid sequence SEQ ID N° 2, or
b) the amino acid sequence containing the amino acids included between positions 24 and 188 of the amino acid sequence SEQ ID N° 2;

it being understood that each one of the amino acid sequences under a) and b) contains at least one of the following coding SNPs: A6D, M171I.

**18.** Polypeptide according to any one of claims 15 to 17 **characterized in that** each one of the amino acid sequences under a) and b) contains a single coding SNP selected from the group consisting of: A6D and M171I.

**19.** Process for obtaining an immunospecific antibody, **characterized in that** it is obtained by immunization of an animal with a polypeptide according to any one of claims 15 to 18.

**20.** Immunospecific antibody for a polypeptide according to any one of claims 15 to 18.

**21.** Process for the identification of an agent activating or inhibiting a polypeptide according to any one of claims 15 to 18, comprising :

a) the preparation of a recombinant vector comprising a polynucleotide according to any one of claims 1 to 4 and 6 to 8,
b) the preparation of host cells comprising a recombinant vector according to a),
c) the contacting of the host cells according to b) with an agent to be tested, and
d) the determination of the activating or inhibiting effect generated by the agent to be tested.

**22.** Process for the identification of an agent activated or inhibited by a polypeptide according to any one of claims 15 to 18, comprising:

a) the preparation of a recombinant vector comprising a polynucleotide according to any one of claims 1 to 4 and 6 to 8,
b) the preparation of host cells comprising a recombinant vector according to a),
c) the contacting of the host cells according to b) with an agent to be tested, and
b) the determination of the activating or inhibiting effect generated by the polypeptide on the agent to be tested.

**23.** Process for analyzing the biological characteristics of a polynucleotide according to the invention and/or of a polypeptide according to the invention in a subject, comprising at least one of the following:

a) Determining the presence or the absence of a polynucleotide according to any one of claims 1 to 8 in the genome of a subject,
b) Determining the level of expression of a polynucleotide according to any one of claims 1 to 8 in a subject,
c) Determining the presence or the absence of a polypeptide according to any one of claims 15 to 18 in a subject,
d) Determining the concentration of a polypeptide according to any one of claims 15 to 18 in a subject, and/or
e) Determining the functionality of a polypeptide according to any one of claims 15 to 18 in a subject.

**24.** Medicament comprising by way of active agent at least one polypeptide according to any one of claims 15 to 18.

**25.** Use of a polypeptide according to any one of claims 15 to 18, for the preparation of a medicament intended for the prevention or the treatment of one of the diseases selected from the group consisting of cancers and tumors, cardiovascular diseases, metabolic diseases, infectious diseases, diseases of the central nervous system, immunologically and auto-immunologically related diseases, healing of wound, disorders connected with chemotherapy treatment, anemia in dialyzed patient, osteoporosis, gastrointestinal disorders, venereal diseases.

**26.** Use of a polypeptide according to any one of claims 15 to 18, for the preparation of a medicament intended for the prevention or the treatment of one of the diseases selected from the group consisting of carcinomas comprising metastasized renal carcinomas, melanomas, lymphomas comprising follicular lymphomas and cutaneous T cell lymphoma, leukemias comprising tricholeucocyte leukemia and chronic myeloid leukemia, cancers of the liver, neck, head and kidneys, multiple myelomas, carcinoid tumors and tumors that appear following an immune deficiency comprising Kaposi's sarcoma in the case of AIDS,cardiovascular diseases, obesity, viral infections comprising chronic hepatitis B and C and HIV/AIDS, and infectious pneumonias, Alzheimer's disease, Parkinson's disease, multiple sclerosis, schizophrenia and depression, the rejection of tissue or organ grafts, allergies, asthma, psoriasis and rheumatoid arthritis, healing of wounds, disorders connected with chemotherapy treatment, anemia in dialyzed patient, osteoporosis, Crohn's disease and ulcerative colitis, and genital warts.

**27.** Medicament comprising by way of active agent at least one polynucleotide according to any one of claims 1 to 4 and 6 to 8, a recombinant vector according to claim 12, a host cell according to claim 13 and/or an antibody according to claim 20.

**28.** Use of a polynucleotide according to any one of claims 1 to 4 and 6 to 8, a recombinant vector according to claim 12, a host cell according to claim 13 and/or an antibody according to claim 20, for the preparation of a medicament intended for the prevention or the treatment of one of the diseases selected from the group consisting of cancers and tumors, cardiovascular diseases, metabolic diseases, infectious diseases, diseases of the central nervous system, immunologically and auto-immunologically related diseases, healing of wound, disorders connected with chemotherapy treatment, anemia in dialyzed patient, osteoporosis, gastrointestinal disorders, venereal diseases.

**29.** Use of a polynucleotide according to any one of claims 1 to 4 and 6 to 8, a recombinant vector according to claim 12, a host cell according to claim 13 and/or an antibody according to claim 20, for the preparation of a medicament intended for the prevention or the treatment of one of the diseases selected from the group consisting of carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,cardiovascular diseases, obesity, viral infections comprising chronic hepatitis B and C and HIV/AIDS, and infectious pneumonias, Alzheimer's disease, Parkinson's disease, multiple sclerosis, schizophrenia and depression, the rejection of tissue or organ grafts, allergies, asthma, psoriasis and rheumatoid arthritis, healing of wounds, disorders connected with chemotherapy treatment, anemia in dialyzed patient, osteoporosis, Crohn's disease and ulcerative colitis, and genital warts.

**30.** Pharmaceutical composition containing by way of active agent at least one polypeptide according to any one of claims 15 to 18, all or part of a polynucleotide according to any one of claims 1 to 4 and 6 to 8, a recombinant vector according to claim 12, a host cell according to claim 13 and/or an antibody according to claim 20, as well as a pharmaceutically acceptable excipient.

**31.** Diagnostic composition containing by way of active agent at least one polypeptide according to any one of claims 15 to 18, all or part of a polynucleotide according to any one of claims 1 to 4 and 6 to 8, a recombinant vector according to claim 12, a host cell according to claim 13 and/or an antibody according to claim 20.

**32.** Use:

a) of a therapeutically effective quantity of a polypeptide according to any one of claims 15 to 18, and/or
b) of a polynucleotide according to any one of claims 1 to 8, and/or
c) of a host cell according to claim 13, this cell coming from the subject to be treated;
in order to prepare a medicament intended to increase the expression or the activity in a subject, of a polypeptide according to any one of claims 15 to 18.

**33.** Use:

a) of a therapeutically effective quantity of an antibody according to claim 20, and/or
b) of a polynucleotide permitting inhibition of the expression of a polynucleotide according to any one of claims 1 to 8;
in order to prepare a medicament intended to decrease the expression or the activity, in a subject, of a polypeptide according to any one of claims 15 to 18.

**34.** Use of a IFNα-2 protein for the preparation of a medicament for the prevention or the treatment of a patient having a disorder or a disease caused by a IFNα-2 variant linked to the presence in the genome of said patient of a nucleotide sequence having at least 95% identity with the nucleotide sequence SEQ ID N° 1, provided that said nucleotide sequence comprises one of the following SNPs: 96-100del(aattt), t110c, 139-144del(acttta), t338a, t363c, c427t, c527a, g1023a, c1047t.

**35.** Method for determining statistically relevant associations between: at least one of the following SNPs: 96-100del (aattt), t110c, 139-144del(acttta), t338a, t363c, c427t, c527a, g1023a, c1047t, in a polynucleotide of the IFNα-2 gene, and a disease or a resistance to a disease comprising:

a) genotyping a group of individuals,
b) determining the distribution of said disease or resistance to disease within said group of individuals,
c) comparing the genotype data with the distribution of said disease or resistance to disease, and
d) analyzing said comparison for statistically relevant associations.

**36.** Use of at least one of the following SNPs: 96-100del(aattt), t110c, 139-144del(acttta), t338a, t363c, c427t, c527a, g1023a, c1047t, in a polynucleotide of the IFNα-2 gene, for developing diagnostic/prognostic kits for a disease or a resistance to a disease.

**37.** A new compound having a biological activity substantially similar or lower in comparison to that of the M171I mutated IFNα2 gene product

**38.** A compound according to claim 37, wherein said biological activity being evaluated, for example, by measuring cellular antiproliferative activity on Daudi Burkitt's cell line or signal transduction capacity.

**39.** A new compound having a cellular antiproliferative activity on Daudi Burkitt's cell line at least 15 times lower than that of the natural wild-type IFNα-2.

**40.** A new compound having a signal transduction capacity on MCF7 cells at least 10 times lower than that of the natural wild-type IFNα-2.

**41.** Use of a polypeptide according to any one of claims 15 to 18, containing the M171I SNP, for the identification of a compound according to any one of claims 37 to 40.

**42.** Process for the identification of a compound according to any one of claims 37 to 40, comprising the following steps:

a) Determining the biological activity, such as the cellular antiproliferative activity on Daudi Burkitt's cell line or signal transduction capacity,
b) Comparing the activity determined in step a) of the compound to be tested, with the activity of M171I mutated IFNα2 gene product, and
b) Determining, on the basis of the comparison carried out in step b), whether the compound to be tested has a substantially similar or lower activity compared to that of the M171I mutated IFNα2 gene product.

**43.** Process according to claim 42, **characterized in that** the compound to be tested is previously identified from synthetic peptide combinatorial libraries, high-throughput screening, or designed by computer-aided drug design so as to have the same three-dimensional structure and/or chemical effect as that of the polypeptide of M171I IFNα2 gene product.

**44.** Use of a compound according to any one of claims 37 to 40, for the preparation of a medicament intended for the prevention or the treatment of one of the diseases selected from the group consisting of cancers and tumors, cardiovascular diseases, metabolic diseases, infectious diseases, diseases of the central nervous system, immunologically and auto-immunologically related diseases, healing of wound, disorders connected with chemotherapy treatment, anemia in dialyzed patient, osteoporosis, gastrointestinal disorders, venereal diseases.

**45.** Use of a compound according to any one of claims 37 to 40, for the preparation of a medicament intended for the prevention or the treatment of one of the diseases selected from the group consisting of carcinomas comprising metastasized renal carcinomas, melanomas, lymphomas comprising follicular lymphomas and cutaneous T cell lymphoma, leukemias comprising hairy-cell leukemia, chronic lymphocytic leukemia and chronic myeloid leukemia, cancers of the liver, neck, head and kidneys, multiple myelomas, carcinoid tumors and tumors that appear following an immune deficiency comprising Kaposi's sarcoma in the case of AIDS,cardiovascular diseases, obesity, viral infections comprising chronic hepatitis B and C and HIV/AIDS, and infectious pneumonias, Alzheimer's disease, Parkinson's disease, multiple sclerosis, schizophrenia and depression, the rejection of tissue or organ grafts, allergies, asthma, psoriasis and rheumatoid arthritis, healing of wounds, disorders connected with chemotherapy treatment, anemia in dialyzed patient, osteoporosis, Crohn's disease and ulcerative colitis, and genital warts.

Figure 1

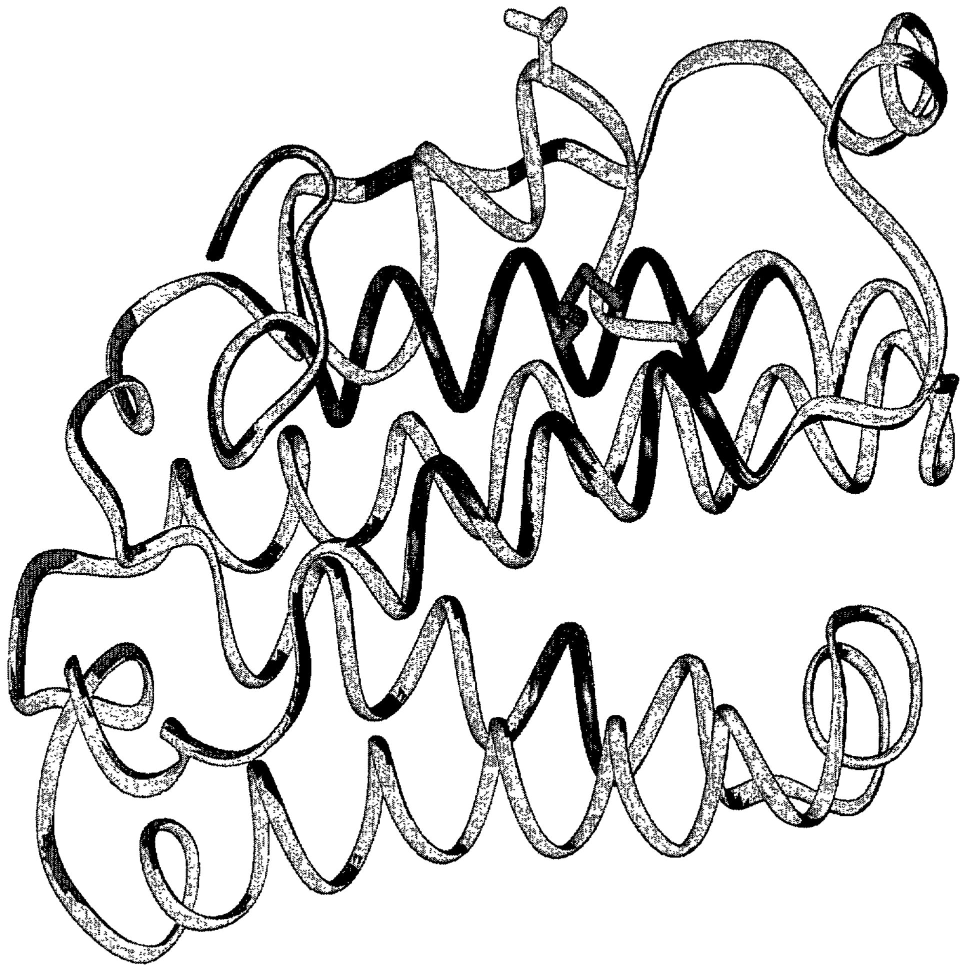

Figure 2

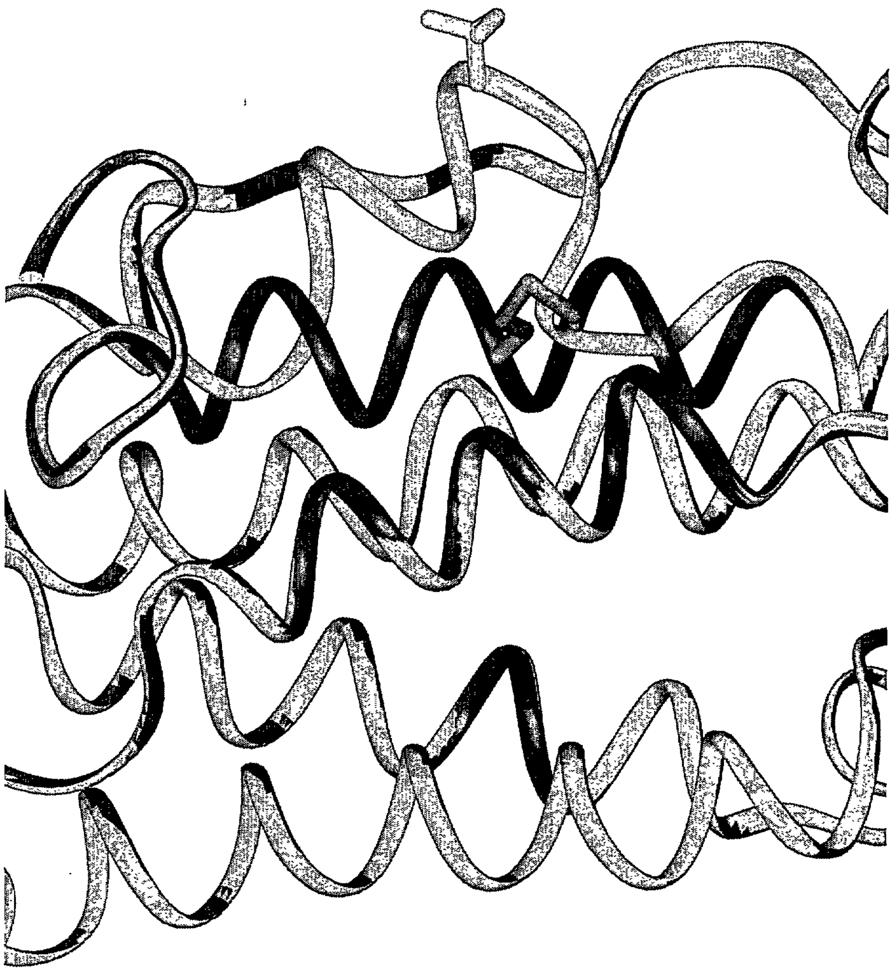

Figure 3

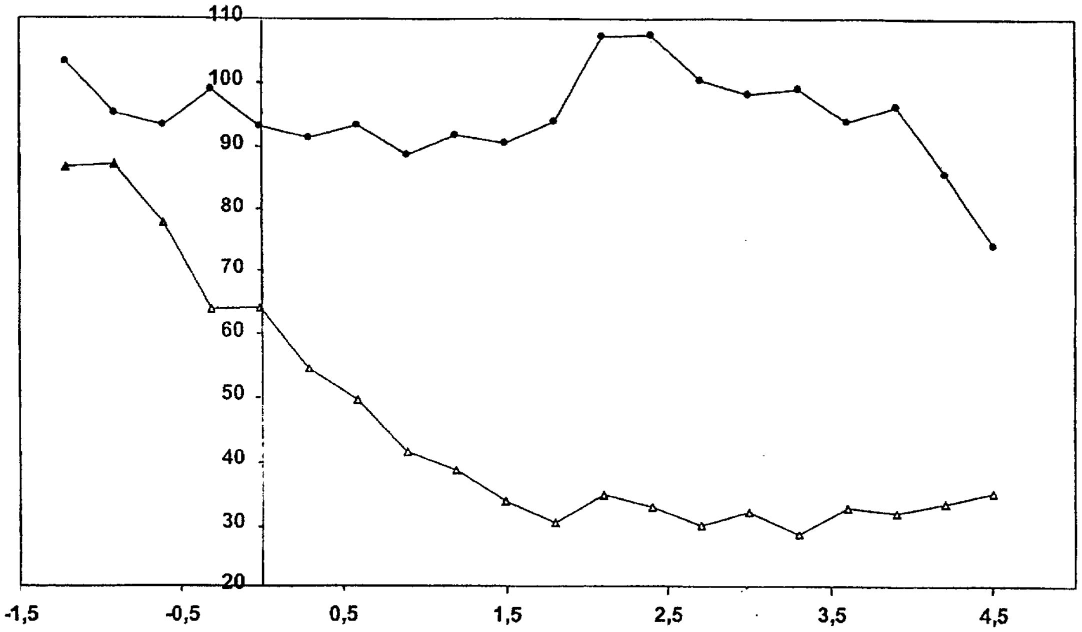

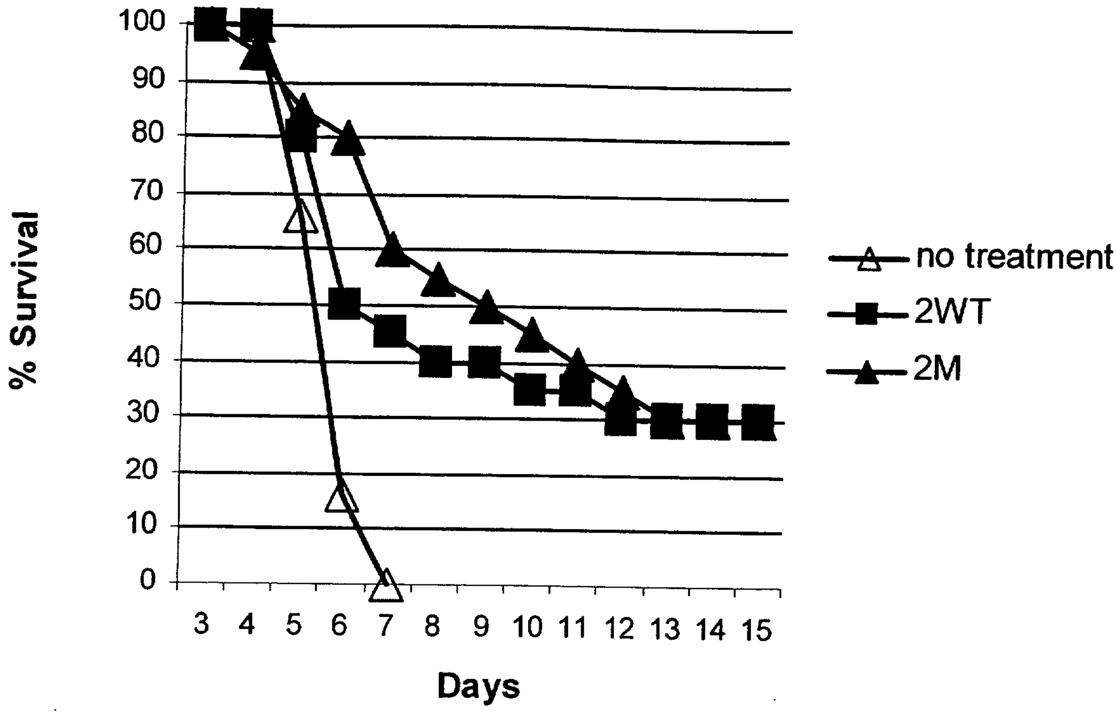
% Survival
100
90
80
70
60
50
40
30
20
10
0
3 4 5 6 7 8 9 10 11 12 13 14 15
Days
no treatment
2WT
2M

Figure 4

SEQUENCE LISTING

<110> GENODYSSEE

<120> New polynucleotides and polypeptides of the IFNa-2 gene

<130> IFNa-2 ext

<140>
<141>

<150> FR.01 02843
<151> 2001-03-01

<160> 2

<170> PatentIn Ver. 2.1

<210> 1
<211> 1733
<212> DNA
<213> Homo sapiens

<400> 1
```
gcgcctctta tgtacccaca aaaatctatt ttcaaaaaag ttgctctaag aatatagtta 60
tcaagttaag taaaatgtca atagcctttt aatttaattt ttaattgttt tatcattctt 120
tgcaataata aaacattaac tttatacttt ttaatttaat gtatagaata gagatataca 180
taggatatgt aaatagatac acagtgtata tgtgattaaa atataatggg agattcaatc 240
agaaaaaagt ttctaaaaag gctctggggt aaaagaggaa ggaaacaata atgaaaaaaa 300
tgtggtgaga aaaacagctg aaaacccatg taaagagtgt ataaagaaag caaaaagaga 360
agtagaaagt aacacagggg catttggaaa atgtaaacga gtatgttccc tatttaaggc 420
taggcacaaa gcaaggtctt cagagaacct ggagcctaag gtttaggctc acccatttca 480
accagtctag cagcatctgc aacatctaca atggccttga cctttgcttt actggtggcc 540
ctcctggtgc tcagctgcaa gtcaagctgc tctgtgggct gtgatctgcc tcaaacccac 600
agcctgggta gcaggaggac cttgatgctc ctggcacaga tgaggagaat ctctcttttc 660
tcctgcttga aggacagaca tgactttgga tttccccagg aggagtttgg caaccagttc 720
caaaaggctg aaaccatccc tgtcctccat gagatgatcc agcagatctt caatctcttc 780
agcacaaagg actcatctgc tgcttgggat gagaccctcc tagacaaatt ctacactgaa 840
ctctaccagc agctgaatga cctggaagcc tgtgtgatac agggggtggg ggtgacagag 900
actcccctga tgaaggagga ctccattctg gctgtgagga aatacttcca aagaatcact 960
ctctatctga aagagaagaa atacagccct tgtgcctggg aggttgtcag agcagaaatc 1020
atgagatctt tttctttgtc aacaaacttg caagaaagtt taagaagtaa ggaatgaaaa 1080
ctggttcaac atggaaatga ttttcattga ttcgtatgcc agctcacctt tttatgatct 1140
gccatttcaa agactcatgt ttctgctatg accatgacac gatttaaatc ttttcaaatg 1200
tttttaggag tattaatcaa cattgtattc agctcttaag gcactagtcc cttacagagg 1260
accatgctga ctgatccatt atctatttaa atatttttaa aatattattt atttaactat 1320
ttataaaaca acttattttt gttcatatta tgtcatgtgc acctttgcac agtggttaat 1380
gtaataaaat gtgttctttg tatttggtaa atttattttg tgttgttcat tgaacttttg 1440
ctatggaact tttgtacttg tttattcttt aaaatgaaat tccaagccta attgtgcaac 1500
ctgattacag aataactggt acacttcatt tgtccatcaa tattatattc aagatataag 1560
taaaaataaa ctttctgtaa accaagttgt atgttgtact caagataaca gggtgaacct 1620
aacaaataca attctgctct cttgtgtatt tgatttttgt atgaaaaaaa ctaaaaatgg 1680
taatcatact taattatcag ttatggtaaa tggtatgaag agaagaagga acg        1733
```

<210> 2
<211> 188

<212> PRT
<213> Homo sapiens

<400> 2

```
Met Ala Leu Thr Phe Ala Leu Leu Val Ala Leu Leu Val Leu Ser Cys
 1               5                  10                  15

Lys Ser Ser Cys Ser Val Gly Cys Asp Leu Pro Gln Thr His Ser Leu
            20                  25                  30

Gly Ser Arg Arg Thr Leu Met Leu Leu Ala Gln Met Arg Arg Ile Ser
        35                  40                  45

Leu Phe Ser Cys Leu Lys Asp Arg His Asp Phe Gly Phe Pro Gln Glu
    50                  55                  60

Glu Phe Gly Asn Gln Phe Gln Lys Ala Glu Thr Ile Pro Val Leu His
65                  70                  75                  80

Glu Met Ile Gln Gln Ile Phe Asn Leu Phe Ser Thr Lys Asp Ser Ser
                85                  90                  95

Ala Ala Trp Asp Glu Thr Leu Leu Asp Lys Phe Tyr Thr Glu Leu Tyr
            100                 105                 110

Gln Gln Leu Asn Asp Leu Glu Ala Cys Val Ile Gln Gly Val Gly Val
        115                 120                 125

Thr Glu Thr Pro Leu Met Lys Glu Asp Ser Ile Leu Ala Val Arg Lys
    130                 135                 140

Tyr Phe Gln Arg Ile Thr Leu Tyr Leu Lys Glu Lys Lys Tyr Ser Pro
145                 150                 155                 160

Cys Ala Trp Glu Val Val Arg Ala Glu Ile Met Arg Ser Phe Ser Leu
                165                 170                 175

Ser Thr Asn Leu Gln Glu Ser Leu Arg Ser Lys Glu
            180                 185
```